(19) Europäisches Patentamt
European Patent Office
Office européen des brevets

(11) **EP 1 438 072 B1**

(12) **EUROPÄISCHE PATENTSCHRIFT**

(45) Veröffentlichungstag und Bekanntmachung des Hinweises auf die Patenterteilung:
**28.05.2008 Patentblatt 2008/22**

(21) Anmeldenummer: **02767035.5**

(22) Anmeldetag: **21.10.2002**

(51) Int Cl.:
*A61K 47/36* (2006.01)     *A61K 9/48* (2006.01)
*C08L 3/00* (2006.01)

(86) Internationale Anmeldenummer:
**PCT/CH2002/000571**

(87) Internationale Veröffentlichungsnummer:
**WO 2003/035026 (01.05.2003 Gazette 2003/18)**

(54) **NETZWERK AUF POLYSACCHARIDBASIS UND VERFAHREN ZU DESSEN HERSTELLUNG**

POLYSACCHARIDE-BASED NETWORK AND METHOD FOR THE PRODUCTION THEREOF

RESEAU A BASE DE POLYSACCHARIDES ET PROCEDE DE FABRICATION

(84) Benannte Vertragsstaaten:
**AT BE BG CH CY CZ DE DK EE ES FI FR GB GR IE IT LI LU MC NL PT SE SK TR**

(30) Priorität: **23.10.2001 DE 10152125**
**28.03.2002 DE 10214327**

(43) Veröffentlichungstag der Anmeldung:
**21.07.2004 Patentblatt 2004/30**

(73) Patentinhaber: **InnoGEL AG**
**6331 Hünenberg (CH)**

(72) Erfinder:
• **MÜLLER, Rolf**
**CH-8055 Zürich (CH)**

• **INNEREBNER, Frederico**
**CH-8049 Zürich (CH)**
• **SMITH, Paul**
**CH-8006 Zürich (CH)**
• **TERVOORT, Theo, A.**
**CH-8006 Zurich (CH)**

(74) Vertreter: **Frommhold, Joachim**
**Bühler AG**
**Patentabteilung**
**9240 Uzwil (CH)**

(56) Entgegenhaltungen:
**DE-A- 10 022 095     DE-A- 19 852 826**

EP 1 438 072 B1

**Beschreibung**

Die Erfindung bezieht sich auf ein Verfahren zur Herstellung eines Netzwerks auf Polysaccharidbasis, sowie auf das dadurch hergestellte Netzwerk und dessen Verwendungen.

Stand der Technik

Polysaccharide, natürlich nachwachsende Stoffe, insbesondere Stärke, bieten hinsichtlich einer Verwendung als Biokunststoff folgende Vorteile: tiefe Rohstoffkosten, hohe Reinheit der Rohstoffe, breite Verfügbarkeit, weites Spektrum an Sorten mit charakteristischen Eigenschaften, nachwachsender Rohstoff, $CO_2$-Neutralität, einfache Verarbeitbarkeit, gute $O_2$-Barriere-Eigenschaften. Aufgrund dieser Vorteile wurden grosse Anstrengungen unternommen, um beispielsweise auf Basis von Stärke, insbesondere von Thermoplastischer Stärke brauchbare Biokunststoffe herzustellen.

Die Herstellung von Thermoplastischer Stärke wird in den Patentschriften WO 94/28029, US 5362777, US 5382611 und US 5427614 beschrieben. Geschäumte Produkte auf Basis von Thermoplastischer Stärke werden in den Patentschriften WO 96/07693, WO 94/28063, WO 95/04104, US 5801207 und US 5736586, Filme auf Basis von Thermoplastischer Stärke in WO 94/04600, US 5415827 und US 5462980 beansprucht. Diese Lösungen haben die Nachteile gemeinsam, dass Thermoplastische Stärke spröde ist, nur eine geringe Bruchdehnungen und eine beschränkte Festigkeit aufweist und als schwerwiegendsten Nachteil, ausgesprochen wasserempfindlich ist, aus der Atmosphäre Wasser aufnimmt und sich in Wasser auflöst.

Aufgrund dieser Probleme wurde Thermoplastische Stärke mit synthetischen hydrophoben Polymeren zu Blends verarbeitet. Entsprechende Lösungen sind in den Patentschriften US 5360830, US 5569692, US 5384170, WO 96/31561, WO 95/04104, WO 97/48764, US 5280055, US 5314934, US 5844023, US 6348524, US 6277899, US 5874486, US 5569692, US 5412005, US 5334634, US 5292782, US 5262458 und US 5258430 beschrieben. Diese Lösungen, bei denen die Thermoplastische Stärke in einer hydrophoben Matrix eingebettet ist, haben den Nachteil, dass nur noch ein Teil der Produkte aus Stärke besteht und biologisch abbaubar ist. Einige Lösungen verwenden synthetische biologisch abbaubare Polymere wie etwa Polycaprolactone als Matrix-Komponente, welche jedoch deutlich teurer sind als Stärke und ebenfalls beschränkte mechanische und thermische Eigenschaften aufweisen. Weitere Lösungen befassen sich mit der chemischen Modifikation von Stärke, um deren Hydrophilie zu reduzieren. Beispiele sind in den Patentschriften WO 98/06755, WO 99/23118 und US 6117925 beschrieben. Nachteilig hierbei ist, dass die entsprechenden chemischen Modifikationen teuer sind, typische Vorteile von Naturstoffen nur noch beschränkt gelten und damit die Produkte wenig konkurrenzfähig sind.

Die bisher bestehenden Ansätze, um auf Basis von Stärke biologisch abbaubare Kunststoffe zu erhalten, konnten das Potential der Stärke nur sehr beschränkt umsetzen. Die ensprechenden Produkte sind entweder bezüglich ihrer Eigenschaften ungenügend und/oder zu teuer, als dass sie sich auf dem Markt im grösseren Stil hätten durchsetzen können. Daher sind auf Stärke basierende biologisch abbaubare Kunststoffe bisher Nischenprodukte geblieben.

Aufgabenstellung

Die vorliegende Erfindung stellte sich die Aufgabe, auf Basis von Polysacchariden und auf rein physikalischem Wege biologisch abbaubare Kunststoffe herzustellen, die gegenüber Thermoplastischer Stärke und einigen anderen biologisch abbaubaren Polymeren verbesserte mechanische Eigenschaften und eine geringere Wasserempfindlichkeit aufweisen, insbesondere in Wasser nicht mehr löslich sind.

Erfindung

Gegenstand der Erfindung sind Netzwerke auf Polysaccharid basis, Verfahren zu ihrer Herstellung und deren Verwendung gemäss den Ansprüchen 1-31. Zur Lösung dieser Aufgabe wurde von Netzwerken oder Gelen von Polysacchariden ausgegangen. Da mit

$$G_0 \sim RTN_0/V_0$$

der Schubmodul $G_0$ proportional zur Netzwerkdichte $N_0/V_0$ ist (R= Gaskonstante, T= absolute Temperatur), ergibt sich die Möglichkeit zur Verbesserung der Festigkeit von Netzwerken über eine entsprechende Erhöhung der Netzwerkdichte, d.h. über die Anzahl aktiver Netzwerkelemente, beziehungsweise Netzwerk-Verknüpfungspunkte $N_0$ pro Volumeneinheit $V_0$. Da die Netzwerkelemente durch Kristallite gebildet werden und diese gegenüber der amorphen Phase eine sehr beschränkte Wasseraufnahmefähigkeit ausweisen, wird durch eine genügend hohe Netzwerkdichten neben den mechanischen Eigenschaften gleichzeitig auch die Wasserempfindlichkeit verbessert. Durch die Wasserunlöslichkeit der Kristallite bei Raum- und erhöhten Temperaturen ist ausserdem die Wasserunlöslichkeit der Netzwerke bei diesen Temperaturen gegeben. Aufgrund dieses Potentials von Polysaccharid-Netzwerken konzentriert sich die vorliegende Erfindung auf Möglichkeiten zur Maximierung der Netzwerkdichte von Polysaccharid-Gelen einerseits durch die Optimierung von Rezepturen, insbesondere durch Maximierung der Konzentration $C_{PNM}$ von netzwerkfähigen Polysacchariden, andererseits durch verfahrenstechnische Massnahmen, insbesondere durch die verfahrenstechnische Beeinflussung der Netz-

werkbildung bei tiefem Weichmachergehalt $WM_0$.

Bei diesbezüglichen Untersuchungen wurde gefunden, dass eine Netzwerkbildung durch geeignete Verfahrensmassnahmen bei erstaunlich tiefen Weichmachergehalten $WM_0$ tatsächlich möglich ist und die entstehenden Netzwerke durch geeignete Kombinationen von vorliegenden Polysacchariden, welche keine oder nur eine geringe Neigung zur Netzwerkbildung aufweisen, mit netzwerkfähigen Polysacchariden bezüglich ihrer Eigenschaften in einem weiten Bereich beeinflusst werden können.

Insbesondere bezüglich dem Polysaccharid Stärke wurde erstaunlicherweise gefunden, dass amylopektinartige Makromoleküle, die erst nach Lagerungszeiten von Wochen bei tiefen Temperaturen Gele von sehr geringer Festigkeit bilden, welche sich bei Temperaturen von rund 60°C wieder auflösen, zusammen mit amyloseartigen Makromolekülen an der Bildung von festen und sogar hochfesten Gelen beteiligt sind, die erst oberhalb rund 120°C im wässrigen Milieu sich wieder auflösen. Selbst bei einem Anteil von nur 5% amyloseartigen und 95% amylopektinartigen Makromolekülen konnten durch geeignete Verfahrensparameter und Mischungsbedingungen Netzwerke erhalten werden, die in ihren Eigenschaften mit reinen Amylose-Gelen vergleichbar, ihnen bezüglich gewisser Eigenschaften wie der Zähigkeit sogar überlegen sind. Dabei wurden auch transparente Netzwerke oder Gele erhalten, die also auf der Grössenskala der Wellenlänge von sichtbarem Licht homogen, d.h. einphasig sind. Dies ist umso erstaunlicher, als bisher Amylopektin und Amylose als nicht mischbar angesehen wurden. Demgegenüber konnten Mischungen durch Anwendung genügend hoher Schergeschwindigkeiten und insbesondere bei tiefen Weichmachergehalten erhalten werden, während die Entmischung durch geeignete Verfahrensparameter unterdrückt werden konnte.

Da auch Netzwerke von Mischungen von Stärken, die für sich selbst zwar kristalliseren, jedoch keine Netzwerke bilden können, mit Stärken wie Amylopektinen, die für sich selbst weder kristallisieren noch Netzwerke bilden können, erhalten werden konnten, kann davon ausgegangen werden, dass infolge des vorgeschlagenen Verfahrens eine Heterokristallisation, insbesondere eine Heterokristallisation von amylopektinartigen Molekülen mit amyloseartigen Molekülen, an der Netzwerkbildung beteiligt ist.

Das Verfahren kann vereinfacht dadurch charakterisiert werden, dass ein vorliegendes Polysaccharid oder eine Mischung vorliegender Polysaccharide, noch nicht, ganz oder teilweise plastifiziert, bei vergleichsweise niederem Weichmachergehalt mit mindestens einem ganz oder teilweise gelösten netzwerkfähigen Polysaccharid oder mit mindestens einer ganz oder teilweise gelösten Mischung von verschiedenen netzwerkfähigen Polysacchariden mit vergleichsweise hohem Weichmachergehalt molekulardispers gemischt wird. Dies ist eine wesentliche Voraussetzung zur Bildung von angestrebten Netzwerken, beispielsweise von einphasigen Stärke-Gelen. Wichtige Verfahrensmassnahmen sind dabei eine Überhitzung der netzwerkfähigen Polysaccharide, eine nachfolgende Unterkühlung vor dem Mischvorgang, ein teilweises Entfernen von Weichmacher beim oder nach dem Mischvorgang zur Einstellung tiefer Weichmachergehalte, sowie die Unterstützung der Netzwerkbildung durch Keime, die im Verfahren erzeugt oder dem Verfahren als Fremdnukleierungsmittel zugeführt werden. Die Ausbildung der Netzwerkstruktur, insbesondere von einphasigen Gelen, wird durch die Rezeptur, sowie durch die kinetische Kontrolle des Gelierungsvorgangs mittels der entsprechenden Verfahrensparameter ermöglicht.

## Vorliegende Polysaccharide

Als vorliegendes Polysaccharid können Hydrokolloide wie beispielsweise irgendeine Stärke oder Mehl, irgendein Agar, irgendeine Agarose, irgendwelche Carrageenane, irgendwelche Alginate, irgendein Chitosan, irgendwelche Pektine, sowie Mischungen verschiedener solcher Polysaccharide, wie auch Mischungen verschiedener Typen desselben Polysaccharids eingesetzt werden. Die vorliegenden Polysaccharide können gelierfähig sein oder auch nicht. Die Polysaccharide können in einem beliebigen Zustand, physikalisch und/oder chemisch modifiziert dem Verfahren zugeführt werden.

Beispiele für in Frage kommende vorliegende Stärken oder Mehle sind folgenden Ursprungs: Getreide wie Mais, Reis, Weizen, Roggen, Gerste, Hirse, Hafer, Dinkel etc.; Wurzeln und Knollen wie Kartoffel, Süsskartoffel, Tapioka (Cassava), Maranta (Arrowroot) etc.; Hülsenfrüchte und Samen wie Bohnen, Erbsen, Mungo, Lotus etc.. Daneben kommen auch Stärken und Mehle anderen Ursprungs in Frage wie beispielsweise Sago, Yams etc.. Ausserdem kann auch Glycogen eingesetzt werden.

Die Polysaccharide können durch Züchtung oder gentechnische Methoden verändert worden sein wie beispielsweise Waxy-Mais, Waxy-Reis, Waxy-Kartoffel, hochamylosehaltiger Mais, indica Reis, japonica Reis et., sie können durch chemische Verfahren verändert worden sein wie beispielsweise durch Säure-Konvertierung, Pyrokonvertierung, Vernetzung, Acetylierung, Hydroxyethylierung, Hydroxypropylierung, Phosphorylierung, Graft-Reaktionen, Reaktionen mit Amylasen et., sie können durch physikalische Verfahren verändert worden sein wie beispielsweise durch Gelatinisierung (teilweise bis vollständig), Plastifizierung, Inhibierung et., oder sie können durch eine Kombination von Züchtung, genetische Methoden, chemische und physikalische Verfahren verändert worden sein.

Beispiele für veränderte Stärken sind dünnkochende Stärken, kaltwasserlösliche Stärken, pregelatinisierte Stärken, hydroxypropylierte Stärken, Dextrine, Maltodextrine, limit-Dextrine, Oligosaccharide, kationische Stärken, Stärkeether, durch Fraktionierung erhaltene Stärken.

Von besonderem Interesse sind im Fall des Polysaccharids Stärke vorliegende Stärken, deren Amylopektin-Fraktion eine mittlere Kettenlänge CL von mindestens 20, vorzugsweise von mindestens 22, noch bevorzugter von mindestens 24, am bevorzugtesten von mindestens 26 aufweisen.

Weiter sind von besonderem Interesse vorliegende Stärken, deren Amylopektin-Fraktion einen Blue Value (BV) von mindestens 0.10, vorzugsweise von mindestens 0.13, noch bevorzugter von mindestens 0.16, am bevorzugtesten von mindestens 0.18 aufweisen.

Ebenfalls sind von besonderem Interesse vorliegende Stärken, deren Amylopektin-Fraktion eine Jod-Affinität (IA) in g/100g von mindestens 0.4, vorzugsweise von mindestens 0.6, noch bevorzugter von mindestens 0.8, am bevorzugtesten von mindestens 1.0 aufweisen.

Bezüglich des Molekulargewichts $M_w$ (Gewichtsmittel) von vorliegenden Polysacchariden sind von besonderem Interesse Polysaccharide mit einem Gewichtsmittel von mehr als 10'000g/mol, vorzugsweise von mehr als 50'000g/mol, noch bevorzugter von mehr als 100'000g/mol, am bevorzugtesten von mehr als 500'000g/mol.

Netzwerkfähige Polysaccharide

Netzwerkfähige Polysaccharide können Polysaccharid-Hydrokolloide wie beispielsweise Stärken, Agar, Agarosen, Carrageenane, Alginate, Chitosane, Pektine sein, sie lassen sich auf folgende Arten definieren.

1. Entsprechend einer ersten Definition können dies Polysaccharide, insbesondere Stärken oder Mehle sein, die unter geeigneten Bedingungen Gele bilden können. Davon ausgenommen sind Gele wie reine Amylopektin-Gele, die sehr lange Gelierungszeiten (Tage bis Wochen) benötigen und dann nur sehr schwache Gele bilden. Netzwerkfähige Polysaccharide können nativ oder physikalisch und/oder chemisch modifiziert worden sein.

1A. Eine Gruppe von Stärken, die dieser Anforderung genügen, sind native oder modifizierte Stärken mit einem Amylosegehalt von mindestens 10%, vorzugsweise von mindestens 20%, noch bevorzugter von mindestens 30%, am bevorzugtesten von mindestens 50%. Besonders geeignet sind beispielsweise hochamylosehaltige Stärken, insbesondere hochamylosehaltige Maisstärken, die einen Amylosegehalt bis nahezu 100% aufweisen können, Erbsenstärken mit Amylosegehalten von mehr als 25% oder Amylosen beliebigen Ursprungs.

1B. Eine weitere Gruppe von netzwerkfähigen Polysacchariden, kann durch chemischen und/oder enzymatischen Abbau, insbesondere durch Entzweigung erhalten werden. Für den enzymatischen Abbau von Stärken beispielsweise können Amylasen, wie α-Amylase, β-Amylase, Glucoamylase, α-Glucosidase, exo-α-Glucanase, Cyclomalto-dextrin, Glucanotransferase, Pullulanase, Isoamylase, Amylo-1,6-Glucosidase oder eine Kombination dieser Amylasen eingesetzt werden. Als Ausgangsstoffe für den Abbau werden dabei insbesondere Stärken der vorgenannten Gruppe von Stärken eingesetzt. Ein Beispiel von chemischem, nicht-enzymatischem Abbau von Polysacchariden, ist die Hydrolyse mittels Säuren wie etwa Salzsäure.

2. Eine weitere Definition netzwerkfähiger Polysaccharide bezieht sich auf den Verzweigungsgrad $Q_b$ der netzwerkfähigen Polysaccharide wobei der Verzweigungsgrad kleiner ist als 0.01, vorzugsweise kleiner als 0.005, noch bevorzugter kleiner als 0.002 am bevorzugtesten kleiner als 0.001, insbesondere kleiner als 0.0001.

3. Ausserdem werden als netzwerkfähige Polysaccharide auch vorwiegend lineare Polysaccharide bezeichnet, die nach erfolgter Lösung kristallisieren können, in Abwesenheit weiterer Polysaccharide jedoch keine Gele, sondern Dispersionen von Kristalliten bilden. Solche Polysaccharide haben mittlere Polymerisationsgrade DP von typischerweise weniger als 100, sie können jedoch in Anwesenheit von Polysacchariden, die sowohl nicht netzwerkfähig, als auch netzwerkfähig sein können, durch Heterokristallisation Gele bilden. Bezüglich dieses Typs von netzwerkfähigen Polysacchariden sind Polysaccharide von Interesse, die eine mittlere Kettenlänge CL oder einen mittleren Polymerisationsgrad von mindestens 10, vorzugsweise von mindestens 20, noch bevorzugter von mindestens 30, am bevorzugtesten von mindestens 50 aufweisen. Im Falle von Stärken kann eine solche netzwerkfähige Stärke beispielsweise ein entzweigtes Maltodextrin sein, das für sich keine Gele bilden kann, jedoch mit einem Amylopektin Gele bildet, die Amylose-Gelen vergleichbar sind.

4. Netzwerkfähige Polysaccharide können andererseits dadurch charakterisiert werden, dass die Makromoleküle lineare Anteile enthalten, wobei diese linearen Anteile Haupt- oder Seitenketten sein können mit mittleren Polymerisationsgraden DP von mehr als 30, vorzugsweise mehr als 50, am bevorzugtesten mehr als 80, insbesondere von mehr als 100, am insbesondersten von mehr als 140.

5. Ausserdem kann eine weitere Gruppe von netzwerkfähigen Stärken durch Fraktionierung von Amylose-Amylopektin-Mischungen erhalten werden, beispielsweise durch Fraktionierung mittels differentieller Alkoholfällung, wobei die Amylose- und die intermediate Fraktion als netzwerkfähige Stärke eingesetzt werden kann.

Erfindungsgemäss werden als netzwerkfähiges Polysaccharid Polysaccharide bezeichnet, welche mindestens eine der Bedingungen 1 - 5 erfüllen. Als netzwerkfähiges Polysaccharid werden auch Mischungen bezeichnet, wobei die Komponenten und/oder die Mischung mindestens eine der obigen Bedingungen erfüllen.

Es wird darauf hingewiesen, dass in bestimmten Fällen vorliegende Polysaccharide und netzwerkfähige Polysaccharide stofflich identisch sein können, da im Prinzip jedes netzwerkfähige Polysaccharid auch als vorliegendes Polysaccharid verwendet werden kann. Der Unterschied zwischen vorliegendem Polysaccharid und netzwerkfähigem Polysaccharid ist daher nicht in allen Fällen stofflicher Art, vielmehr müssen die Begriffe auch in Zusammenhang mit dem Verfahren definiert werden. Netzwerkfähige Polysaccharide werden in einer Weise behandelt, dass deren Potential zur Bildung von Netzwerken optimal freigesetzt wird, während dies bei vorliegenden Polysacchariden ohne geeigneten Löse- und Unterkühlungsvorgang nicht der Fall sein muss.

Verfahren

1. Lösen und Unterkühlen netzwerkfähiger Polysaccharide

Erst durch geeignetes Lösen von netzwerkfähigen Polysacchariden wird ihr Potential zur Bildung von Netzwerken freigesetzt. Durch Plastifizierung, wie dies beispielsweise bei der Herstellung von Thermoplastischer Stärke üblich ist, ist dies höchstens teilweise gewährleistet oder sind bei tiefer Weichmacherkonzentration sehr hohe Temperaturen notwendig, die dann zu einem starken thermischen Abbau führen. Der Lösevorgang von netzwerkfähigen Polysacchariden wie auch von netzwerkfähigen Stärken ist ein vielstufiger und komplexer Vorgang. Der Lösevorgang erstreckt sich üblicherweise über einen Temperaturbereich von einigen °C, wobei sukzessive Ordnungsstrukturen aufgelöst werden, bis eine vollständige Lösung erfolgt ist. Der Temperaturbereich zeigt ausserdem eine starke Abhängigkeit von der Konzentration. Der Lösevorgang ist weiterhin auch abhängig von einer mechanischen Beanspruchung durch Scherung, wodurch das Lösen bei tieferer Temperatur erfolgen kann, sowie vom Druck, der Lösungszeit, der Aufheizgeschwindigkeit und dem pH. Es kann durchaus sinnvoll sein, wenn die Lösung nicht vollständig ist, also Reststrukturen noch erhalten bleiben, die dann als Keime bei der nachfolgenden Netzwerkbildung wirken können. Allerdings wird eine Überhitzung der Lösung bevorzugt, wobei eine vollständige Lösung, somit eine Standardisierung erhalten wird. Unter Überhitzung wird der Vorgang verstanden, wobei eine Temperatur angewendet wird, die höher liegt als die Lösungstemperatur.

Die für die Netzwerkbildung wirksamen Keime können danach in grösserer Anzahl und Effektivität durch eine definierte Unterkühlung erhalten werden, wodurch sich sehr fein strukturierte Netzwerke mit entsprechend guten mechanischen Eigenschaften herstellen lassen, insbesondere auch einphasige Gele. Die verschiedenen Parameter des Löse- und Unterkühlungsvorgangs sind deshalb für die Struktur und Eigenschaften der nach dem Mischvorgang mit vorliegenden Polysacchariden sich ergebenen Gele von zentraler Bedeutung. Zur Beeinflussung der Keimbildung können überdies auch Fremdkeime bzw. Fremdnukleierungsmittel eingesetzt werden. Besonders hohe Keimzahlen und entsprechend feine und vorteilhafte Gelstrukturen können durch die Anwendung von Druck und Druckwellen, beispielsweise durch Ultraschall vor dem Mischen mit vorliegenden Polysacchariden erhalten werden.

Besonders wirksame Keime werden auch dadurch erhalten, dass zusammen mit netzwerkfähigen Polysacchariden ein Anteil eines Polysaccharids gelöst wird, das für sich alleine kaum Gele bildet, wobei sich bei der anschliessenden Unterkühlung gemischte Keime ergeben, die für einphasige Gele von besonderer Bedeutung sind. Ausserdem sind dadurch grössere Unterkühlungen und entsprechend höhere Keimzahlen anwendbar.

Verschiedene netzwerkfähige Polysaccharide können zusammen gelöst, unterkühlt und dann vorliegenden Polysacchariden zugemischt werden. Da jedoch verschiedene netzwerkfähige Polysaccharide eine unterschiedliche Löse- und Keimbildungscharakteristik aufweisen, ist es oft sinnvoll, sie getrennt aufzubereiten und getrennt dem Mischvorgang zuzuführen.

Da netzwerkfähige Polysaccharide Lipide und Proteine enthalten können, welche mit den linearen Anteilen der netzwerkfähigen Polysaccharide Komplexe bilden und damit diese linearen Anteile für die Netzwerkbildung nicht mehr zur Verfügung stehen, ist es bei höheren Lipid- und Proteinanteilen angezeigt, diese Stoffe vorgängig durch Extraktion zu entfernen. Sie können jedoch auch nach dem Lösevorgang, bei der nachfolgenden Unterkühlung, wobei sie aus der Lösung ausfallen, durch Filtration aus dem Prozess entfernt werden.

Die Parameter des Löse- und Unterkühlungsvorgangs sind wie folgt:

1. Der Weichmachergehalt $WM_d$ in Gew.% der netzwerkfähigen Polysaccharide in Schritt d) und e) liegt im Bereich 40% - 99%, vorzugsweise im Bereich 40% - 94%, noch bevorzugter im Bereich 40% - 87%, insbesondere im Bereich 40% - 80%, am bevorzugtesten im Bereich 40% - 70%. Vergleichsweise niedere Weichmachergehalte $WM_d$ beziehungsweise hohe Konzentrationen $C_{PN}$ von netzwerkfähigen Polysacchariden im zweiten Fluid sind von Bedeutung, um tiefe Weichmachergehalte $WM_0$ und $WM_2$ und $WM_3$, hohe Netzwerkdichten $N_0/V_0$ und damit hohe Festigkeiten des Netzwerks zu erhalten.

2. Der Druck p während dem Überführen in Schritt d) und e) ist identisch mit dem Wasserdampfdruck $p_w(T)$ bei der jeweiligen Temperatur, vorzugsweise maximal 2 $p_w(T)$, noch bevorzugter maximal 5 $p_w(T)$, insbesondere maximal 10 $p_w(T)$, am bevorzugtesten maximal 100 $p_w(T)$. Hohe Drücke sind insbesondere in Schritt e) zur Einstellung hoher Keimzahlen $Z_K$ im dritten Fluid von Bedeutung.

3. Die Überhitzungs-Temperatur $T_{L\ddot{u}}$ in Schritt d) beträgt mindestens 80°C, vorzugsweise mindestens 120°C, noch bevorzugter mindestens 160°C, insbesondere mindestens 180°C, am bevorzugtesten mindestens 200°C. Sie kann maximal bis 260°C betragen, wobei solch hohe Temperaturen nur für sehr kurze Zeiten zur Anwendung kommen können. Hohe Temperaturen $T_{L\ddot{u}}$ wirken sich stabilisierend auf die Lösung aus, d.h. je höher $T_{L\ddot{u}}$, bei umso tieferer Temperatur bleibt die Lösung danach noch stabil, wodurch der Verfahrensspielraum vergrössert wird. Ausserdem ermöglicht eine Überhitzung bei geeigneter Prozessführung die Einstellung hoher Keimzahlen $Z_K$ bei der Unterkühlung in Schritt e). Eine weitere Möglichkeit der Stabilisierung der Lösungen kann durch Zugabe von Keimstabilisatoren erreicht werden.

4. Die Zeitdauer $\Delta t_d$ des Überführens in Schritt d) beträgt maximal 7 min, vorzugsweise maximal 3 min, noch bevorzugter maximal 1 min, insbesondere maximal 0.5 min, am bevorzugtesten maximal 0.2 min, minimal beträgt die Zeitdauer 5sec. Kurze Überführungszeiten sind insbesondere bei hohen Temperaturen $T_{L\ddot{u}}$ zur Unterdrückung von thermischem Abbau von Bedeutung.

5. Die Aufheizgeschwindigkeit $v_d$ beim Überführen in Schritt d) beträgt mindestens 1°C/min, vorzugsweise mindestens 10°C/min, noch bevorzugter mindestens 50°C/min, insbesondere mindestens 100°C/min, am bevorzugtesten mindestens 200°C/min, maximal beträgt die Aufheizgeschwindigkeit etwa 300°C/min. Hohe Aufheizgeschwindigkeiten sind insbesondere bei hohen Konzentrationen $C_{PN}$, bei hohen Molekulargewichten dieser Polysaccharide, bei hoher Temperatur $T_{L\ddot{u}}$ in Schritt d) und zur Unterdrückung eines thermischen Abbaus von netzwerkfähigen Polysacchariden wichtig.

6. Die Temperatur $T_{L1}$ in Schritt e) beträgt maximal 0.9 $T_{L\ddot{u}}$, vorzugsweise maximal 130°C, noch bevorzugter maximal 100°C, insbesondere maximal 70°C, am bevorzugtesten maximal 30°C. Die minimale Temperatur liegt bei etwa 0°C. Tiefe Temperaturen $T_{L1}$ sind zur Einstellung hoher Unterkühlungen und zur Einstellung hoher Keimzahlen von Bedeutung.

7. Die Zeitdauer $\Delta t_e$ des Überführens in Schritt e) beträgt maximal 7 min, vorzugsweise maximal 3 min, noch bevorzugter maximal 1 min, insbesondere maximal 0.5min, am bevorzugtesten maximal 0.2 min, die kürzesten Zeiten liegen bei etwa 5sec. Kurze Zeiten sind notwendig, um hohe Unterkühlungen $\Delta T_{LU}$ und damit hohe Keimzahlen $Z_K$ zu erhalten, ohne dass dabei eine Netzwerkbildung oder Kristallisation der netzwerkfähigen Polysaccharide einsetzt.

8. Die Abkühlgeschwindigkeit $v_e$ deim Überführen in Schritt e) beträgt mindestens 5°C/min, vorzugsweise mindestens 30°C/min, noch bevorzugter mindestens 70°C/min, insbesondere mindestens 110°C/min, am bevorzugtesten mindestens 200°C/min, maximal beträgt die Abkühlungsgeschwindigkeit etwa 300°C/min. Durch hohe Abkühlungsgeschwindigkeiten kann eine hohe Keimzahl im zweiten Fluid $Z_K$ erreicht werden, ohne dass dabei eine Netzwerkbildung oder Kristallisation der netzwerkfähigen Polysaccharide einsetzt.

9. Der pH in den Schritten d) und e) liegt für Stärke im Bereich 5 - 12, vorzugsweise im Bereich 6 -12, noch bevorzugter im Bereich 7 bis 12. Ein erhöhter pH erleichtert die Löslichkeit von netzwerkfähiger Stärke. Gegebenenfalls wird der pH der Gesamtmischung in Schritt g) durch Zugabe einer Säure oder Base auf den gewünschten Wert, vorzugsweise auf pH 6 - 8 eingestellt.

10. Die Schergeschwindigkeit $G_d$ in den Schritten d) und/oder e) und f) beträgt mindestens 10/s, vorzugsweise mindestens 100/s, noch bevorzugter mindestens 1000/s, insbesondere mindestens 10'000/s, am bevorzugtesten mindestens 50'000/s. Maximale Schergeschwindigkeiten liegen bei rund 100'000/s. Durch hohe Schergeschwindigkeiten kann in Schritt d) die Löslichkeit insbesondere von netzwerkfähigen Polysacchariden mit hohem Molekulargewicht deutlich verbessert werden und können somit höhere Konzentrationen $C_{PN}$ verarbeitet werden. In Schritt e) verhindern hohe Schwergeschwindigkeiten eine vorzeitige Netzwerkbildung.

Die verschiedenen Parameter des Lösungs- und Unterkühlungsvorgangs werden entsprechend den vorstehenden Angaben dahingehend optimiert, um bei hohen Konzentrationen $C_{PN}$, hohen Temperaturen $T_{L\ddot{u}}$ und geringfügigem thermischem Abbau stabile Lösungen zu erhalten, die zu tiefen Temperaturen unterkühlt werden können, dabei hohe Keimzahlen $Z_K$ entwickeln, dabei jedoch weder auskristallisieren noch Netzwerkbildung zeigen. Dies ist eine Voraussetzung, um nach erfolgter Mischung mit vorliegenden Polysacchariden Netzwerke mit hohen Netzwerkdichten und Festigkeiten zu erhalten.

Entsprechend den obigen Bedingungen 1 bis 10 behandelte netzwerkfähige Polysaccharide werden anschliessend mit vorliegenden Polysacchariden gemischt, um Netzwerke zu erhalten, wobei sowohl netzwerkfähige Polysaccharide als auch vorliegende Polysaccharide einen Beitrag zum entstehenden Netzwerk leisten. Andererseits ergeben die nach den Bedingungen 1 bis 10 behandelten netzwerkfähigen Polysaccharide auch ohne nachfolgende Mischung bereits neuartige Netzwerke oder Gele, welche sich für bestimmte Anwendungen eignen und dabei neue Produkteigenschaften ermöglichen.

Nachdem ein netzwerkfähiges Polysaccharid oder eine Mischung von Polysacchariden entsprechend obigen Bedingungen gelöst und gegebenenfalls unterkühlt worden sind, können sie direkt mit vorliegenden Polysacchariden gemischt werden oder aber zwei oder mehrere Lösungen werden zuerst zusammengeführt, gemischt und dann vorliegenden Polysacchariden zugeführt. Es ist in bestimmten Fällen auch möglich, aufbereitete Polysaccharide in jeweils verschiedene erste Fluide von vorliegenden Stärken einzumischen und diese Mischungen dann zu einer Gesamtmischung zu vereinigen.

2. Mischen von vorliegenden Polysacchariden mit netzwerkfähigen Polysacchariden

Insbesondere um einphasige Gele zu erhalten, ist eine molekulardisperse Mischung von vorliegenden Polysacchariden und netzwerkfähigen Polysacchariden eine wesentliche Voraussetzung. Solche Mischungen können durch Anwendung von Scherung und hohen Schergeschwindigkeiten erhalten werden. Ist eine molekulardisperse oder nahezu molekulardisperse Mischung eingestellt worden, kann eine Phasenseparation durch die kinetische Kontrolle des Prozesses eingeschränkt oder ganz verhindert werden. Dies bedeutet eine entsprechende Kontrolle der Abkühlungsgeschwindigkeit, wobei der einphasige thermodynamisch metastabile Zustand eingefroren werden kann.

1. Der Weichmachergehalt $WM_1$ in Gew.% der vorliegenden Polysaccharide in Schritt c) vor dem Zuführen der netzwerkfähigen Polysaccharide beträgt 5% - 90%, vorzugsweise 5% - 70%, noch bevorzugter 5% - 60%, insbesondere 5% - 50%, am bevorzugtesten 5% - 45%.

2. Der durchschnittliche Verzweigungsgrad $Q_b$ der Polysaccharid-Mischung in Schritt g) liegt infolge der Mischung mit meist deutlich stärker verzweigten vorliegenden Polysacchariden üblicherweise höher als der durchschnittliche Verzweigungsgrad der eingesetzten netzwerkfähigen Polysaccharide, $Q_b$ ist kleiner als 0.05, vorzugsweise kleiner 0.02, noch bevorzugter kleiner als 0.006, insbesondere kleiner als 0.003, am bevorzugtesten kleiner als 0.001.

3. Weichmachergehalt $WM_2$ in Gew.% unmittelbar nach Schritt g) ist kleiner als 80%, vorzugsweise kleiner als 60%, noch bevorzugter kleiner als 50%, insbesondere kleiner als 40%, am bevorzugtesten kleiner als 30%. Der minimale Weichmachergehalt $WM_2$ liegt bei 10%.

4. Die Schergeschwindigkeit $G_g$ beim Mischen von erstem Fluid mit zweitem Fluid beträgt mindestens 10/s, vorzugsweise mindestens 100/s, noch bevorzugter mindestens 1000/s, insbesondere mindestens 10'000/s, am bevorzugtesten mindestens 50'000/s. Die maximale Schergeschwindigkeit liegt bei etwa 100'000/s. Durch hohe Schergeschwindigkeiten wird bevorzugt eine mokelulardisperse Mischung der Fluide erreicht, was für hohe resultierende Netzwerkdichten $N_0/V_0$ und insbesondere für einphasige Netzwerke eine Voraussetzung ist. Ausserdem wird durch hohe Schergeschwindigkeiten $G_g$ eine grosse Zahl von möglichst kleinen die Netzwerkelemente bildenden Kristalliten erhalten.

5. Zusätzlich kann die Netzwerkdichte nach erfolgter Netzwerkbildung in der Mischung durch geeignete Fremdnukleierungsmittel erhöht werden. Die Anzahl der bei der Netzwerkbildung wirksamen Keime Z ist dann gegeben durch $Z = Z_K + Z_N$, wobei $Z_K$ die Zahl der Keime im zweiten Fluid und $Z_N$ die Zahl der Fremdkeime ist.

6. Die Konzentration $C_{PNM}$ der entsprechend den Schritten d) bis f) verarbeiteten netzwerkfähigen Polysaccharide in der Mischung von Schritt g) in Gew. % beträgt 1% - 95%, vorzugsweise 2% - 70%, noch bevorzugter 3% bis 50%, insbesondere 3% bis 30%, am bevorzugtesten 3% - 25%. Durch Anwendung hoher Konzentrationen $C_{PN}$ von netzwerkfähigen Stärken im zweiten und dritten Fluid können nach erfolgter Mischung mit vorliegenden Polysacchariden entsprechend hohe Konzentrationen $C_{PNM}$ von netzwerkfähigen Polysacchaariden in der Mischung erhalten werden, wodurch sich hohe Netzwerkdichten $N_0/V_0$ und damit hohe Festigkeiten des Netzwerks einstellen lassen.

In mindestens einem der Schritte a) bis g) kann mindestens ein Weichmacher mindestens teilweise aus dem Prozess entfernt werden, dies ist insbesondere bei Schritt g) wichtig, da durch die Reduktion des Weichmachergehalts die Phasenseparation unterdrückt werden kann, indem die Mobilität der Moleküle eingeschränkt wird.

Um neue Polysaccharid-Netzwerke zu erhalten, wird beispielsweise mit einem thermoplastischen Verfahren vorliegenden Polysacchariden mit Weichmachergehalt $WM_1$, vorzugsweise in einem plastifizierten Zustand (erstes Fluid) sich befindend, bei einer Temperatur $T_1 > T_g$ ein zweites oder drittes, netzwerkfähige Polysaccharide enthaltendes Fluid im ersten Fluid di-

spergiert, vorzugsweise molekulardispers gemischt.

Die Lösung der netzwerkfähigen Polysaccharide, d.h. das zweite oder dritte Fluid wird mit der Temperatur $T_{L1}$ ($T_{L1} > = T_{LM}$) und der Keimzahl $Z_K$ zu vorliegenden Polysacchariden im Schritt f) bei $T_1 > T_g$ zudosiert, beispielsweise durch Einspritzung in ein kontinuierlich arbeitendes Mischaggregat, beziehungsweise im ersten Fluid gelöst, wobei $T_{L1}$ grösser, gleichgross oder im Hinblick auf eine hohe Keimzahl $Z_K$ auch unterhalb $T_1$ liegen kann. In Spezialfällen kann der Weichmacher für das vorliegende Polysaccharid auch nach der Einspritzung des netzwerkfähige Polysaccharide enthaltenden Fluids zugegeben werden. Durch die Zudosierung der Lösung steigt der Weichmachergehalt der Mischung auf $WM_2$, denn üblicherweise ist $WM_1 < 1\text{-}C_{PN}$. Ist das zweite oder dritte Fluid genügend dispergiert, wird der Stabilisator dadurch inaktiviert, denn durch den Mischvorgang sinkt die Konzentration $C_{StaM}$ des Stabilisators in der Mischung gegenüber der Konzentration $C_{Sta}$ des Stabilisators in der Lösung, doch die zuvor entstandenen Keime bleiben bei geeigneter Prozessführung erhalten. Durch Zudosierung von weiterem Weichmacher oder durch Entfernen von Weichmacher mittels Entgasungstechnik während oder nach dem Mischvorgang weist die Mischung von erstem und zweitem oder drittem Fluid am Ende des Mischvorgangs den Weichmachergehalt $WM_3$ auf, der üblicherweise dem Weichmachergehalt $WM_0$ entspricht, der bei der Netzwerkbildung nach erfolgter Formgebung gegenwärtig ist.

Die Temperatur der Mischung $T_M$, ändert sich während des Mischvorgangs abhängig vom Weichmachergehalt und von verfahrensspezifischen Parametern. Am Ende des Mischvorgangs liegt die Temperatur der Mischung bei $T_3$. In den meisten Fällen wird so verfahren, dass während der Verarbeitung der Mischung keine Netzwerkbildung stattfindet, weil dann ein sich entwickelndes Netzwerk geschädigt würde, d.h. die Temperatur $T_K$, da die Netzwerkbildung einsetzt, soll vor der Formgebung nicht oder höchstens kurzzeitig unterschritten werden ($T_M > T_K$).

Dies kann durch eine geeignete Wahl der Parameter $WM_1$, $WM_2$, $WM_3$, der Konzentration der gelösten netzwerkfähigen Polysaccharide $C_{PNM}$ in der Mischung, durch die Überhitzung bei $T_{\ddot{U}}$, die Unterkühlung $\Delta T_{LU}$ und die Keimstabilisatorkonzentration $C_{Sta}$ wodurch die Keimzahl $Z_K$ im dritten Fluid gegeben ist, durch die Zahl $Z_N$ der Fremdkeime in der Mischung, sowie durch die Kontrolle der Temperaturen $T_1$ und $T_M$ und des Drucks während der Verarbeitung erreicht werden. Die Unterdrückung der Netzwerkbildung ist insbesondere bei der Anwendung von hohen Konzentrationen $C_{PNM}$ und bei mittleren bis hohen Weichmachergehalten von Bedeutung. Bei tiefen Weichmachergehalten ist die Unterdrückung der vorzeitigen Netzwerkbildung während der Verarbeitung weniger problematisch, weil die Mobilität der netzwerkfähigen Polysaccharide dann stark eingeschränkt ist.

In gewissen Fällen allerdings wird eine Netzwerkbildung, insbesondere eine partielle Netzwerkbildung vor der endgültigen Formgebung angestrebt, z.B. dann, wenn die Mischung zu Fasern geformt werden soll. Eine partielle Netzwerkbildung ermöglicht dann besonders hohe Orientierungen und entsprechend hohe Festigkeiten der Fasern beim Gel-Spinnverfahren, wobei das Netzwerk im Schritt h1) und/ oder h2) gereckt wird.

Die Schritte a) bis h), beziehungsweise a) bis h1) und a) bis h2) werden vorzugsweise zumindest in Teilbereichen kontinuierlich durchgeführt, wobei die geeignete Verfahrenszone der Prozessraum mindestens eines Mischers ist und die Schritte a) bis h) kontinuierlich in aufeinanderfolgenden Abschnitten des mindestens einen Mischers und die Schritte h1) und h) in einer dem mindestens einen Mischer nachgeschalteten Formgebungseinheit stattfinden. Der mindestens eine Mischer kann ein Einschnecken- oder ein Zweischnecken- oder ein Mehrschnecken- oder ein Ringextruder oder ein Kokneter oder ein statischer Mischer oder ein Ystral Mischer oder eine Rührwerkskugelmühle oder eine andere bezüglich Temperatur, Druck und Scherung regelbare Verfahrensstrecke sein.

3. Formgebung und Netzwerkbildung

Nachdem die Dispergierung der netzwerkfähigen Polysaccharide im ersten Fluid stattgefunden hat, die Zuschlagstoffe eingemischt sind und der Weichmachergehalt $WM_3$ eingestellt ist, die Mischung die Temperatur $T_3$ erreicht hat und der Formgebung zugeführt worden ist, setzt die zu diesem Zeitpunkt erwünschte Netzwerkbildung beim nachfolgenden kontrollierten Abkühlvorgang ein. Für die Formgebung können die in der Kunststofftechnik üblichen Verfahren eingesetzt werden.

Bei tiefen Weichmachergehalten, die insbesondere für die Herstellung von hochfesten Polysaccharid-Gelen notwendig sind, ist die Mobilität der netzwerkfähigen Polysaccharide so sehr eingeschränkt, dass die Netzwerkbildung beim an die Formgebung anschliessenden Abkühlvorgang nicht mehr oder nur noch zu einem geringen Teil stattfinden kann. Die Mischung liegt dann bei Raumtemperatur in einem thermodynamisch metastabilen eingefrorenen Zustand vor und infolgedessen ist das dreidimensionale Netzwerk nur geringfügig ausgebildet. Durch die aus der Kombination von Überhitzung und Unterkühlung resultierenden Keime im dritten fluiden Zustand und mittels weiterer Fremdnukleierungsmittel, kann die Grenze des tiefsten Weichmachergehalts $WM_0$, bei dem die Netzwerkbildung noch stattfinden kann, zu tieferen Werten hin gesenkt werden. Mittels einer auf die Formgebung folgenden geeigneten Wärmebehandlung können daher auch Mischungen mit tiefen Weichmachergehalten ein voll entwickeltes Netzwerk ausbilden, das sich durch sehr kleine Kristallite, welche die Vernetzungspunkte des Netzwerks bei hoher Netzwerkdichte bilden, auszeichnet und zu transparenten Filmen führt. Zur Unterstützung der Netzwerkbildung bei tiefen Weichmachergehalten $WM_0$ ist der Einsatz von Ultraschallwellen vorteilhaft.

Von besonderer Bedeutung sind Überhitzung und Unterkühlung und/oder der Einsatz von Fremdnukleierungsmitteln auch dann, wenn die Mischungen durch Spritzguss zu Formkörpern geformt werden. Für ein profitables Verfahren müssen die Abkühlzeiten der Formkörper möglichst kurz sein.

Hochfeste Gele mit tiefen Weichmachergehalten $WM_0$ sind selbst im gequollenen Zustand praktisch transparent, weil die Grösse der Kristallite unterhalb der Wellenlänge des sichtbaren Lichts liegt und die Kristallite das Licht deshalb nicht zu streuen vermögen. Dies ist ein Indiz dafür, dass es durch die getroffenen Massnahmen gelungen ist, die Netzwerkdichte zu erhöhen, indem die Kristallitgrösse verkleinert worden ist. Solche transparente Gele werden als einphasige Gele bezeichnet. Bei höheren Weichmachergehalten bilden sich grössere Kristallite aus, deren Grösse in der Grössenordnung der Wellenlänge des sichtbaren Lichts oder grösser ist, die deshalb das Licht zu streuen vermögen, also nicht transparent sind und eine milchig weisse Farbtönung aufweisen, wie dies bei herkömmlichen Gelen beobachtet werden kann.

Weichmacher

Als Weichmacher können grundsätzlich dieselben Lösungsmittel, Weichmacher und Weichmachermischungen verwendet werden, die entsprechend dem Stand der Technik als Lösungsmittel, Weichmacher und Weichmachermischungen für die entsprechenden Polysaccharide und Hydrokolloid-Polysaccharide eingesetzt werden, bevorzugt werden sie aus folgender Gruppe ausgewählt:

Wasser; Glycerin; Glycerinethoxylat; Polyglycerine; Di-, bis Decaglycerine; Polyglycerinmonoethoxylate; Reaktionsprodukte von Glucose mit Ethylenoxid; Glucosemonoethoxylat; Glucoside; Butylglucosid; Aphamethylglucosid; Maltose, Glucotri- und höhere Glucopolysaccharide, Mono- und Oligosaccharid-Sirupe; Alkohole; Polyalkohole; Butanol; Erythritol; Pentaerythritol; Triethylolpropan; Trimethylolpropan; Triethylpropanmonoethoxylat; Propandiole; Butandiole; Pentandiole; Hexandiole; Hexantriole; Polyvinylalkohole mit 3 bis 20 Monomereinheiten; ganz oder teilweise zu Polyvinylalkoholen hydrolysierte Polyvinylacetate; Trihydroxymethylaminomethane; Aminoalkohole; Fettalkohole; Amine; Hydroxyalkydamin; Ethylendiamin; Amide; Esteramide; Formamid; Säureamide; Sulfoxide; DMSO; quaternäre Ammonium-Verbindungen; Glycol, Ethylenglycol; Ethylendiglycol; Ethylentriglycol; Propylenglycol; Propylendiglycol; Propylentriglycol; Neopentylglycol; Polyethylenglykole; Polypropylenglycol; Polyglycole; Pyrrolidone; 2-Pyrrolidon oder 1-Methyl-2-Pyrrolidon; Caprolactan; Polycaprolactan; Sorbitol; Sorbitolacetat; Sorbitoldiacetat; Sorbitolmonoethoxylat; Sorbitoldipropoxylat; Sorbitoldiethoxylat; Sorbitolhexaethoxylat; Salze von Carboxymethylsorbitol; Aminosorbitol; Maltitol; Mannitol; Mannitolmonoacetat; Mannitolmonoethoxylat; Xylitol; Arabitol; Adonitol; Iditol; Galactitol; Allitol; Säuren; Carboxylsäuren; Ameisensäure; Essigsäure; Bernsteinsäure; Bernsteinsäureanhydrid; Adipinsäure; Milchsäure; Weinsäure; Citronensäure: Apfelsäure; Hydroxybuttersäure; Maleinsäure; Fettsäuren; Dimethylsulfoxid; Harnstoff; chemisch veränderte, insbesondere durch Veresterung erhaltenen Elemente dieser Gruppe; Mischungen von Elementen dieser Gruppe.

Weichmacher oder Weichmachermischungen werden üblicherweise den vorliegenden Polysacchariden in Schritt b) und den netzwerkfähigen Polysacchariden in Schritt d) zugeführt, zusätzlicher Weichmacher kann ausserdem auch in mindestens einem der Schritte a), c), e) f), g) oder h) dem Verfahren zugeführt werde. Die Zuführung von Weichmacher in Schritt b) kann entfallen, wobei dann der Schritt c) ebenfalls entfällt und das entsprechende vorliegende Polysaccharid in Schritt g) gleichzeitig mit dem Mischen zu der Gesamtmischung in ein Fluid überführt, beziehungsweise plastifiziert wird.

Weichmacher kann gegebenenfalls in mindestens einem der Schritte aus dem Verfahren entfernt werden, beispielsweise durch Entgasungstechniken, insbesondere in mindestens einem der Schritte g), h), h1) und h2). Dies ist für die Herstellung von Netzwerken mit niederem Weichmachergehalt $WM_0$ von Bedeutung.

Zusätze

1. Fremdnukleierungsmittel

Fremdnukleierungsmittel können insbesondere bei tiefen Weichmachergehalten $WM_0$ in mindestens einem der Schritte a) bis g) dem Prozess zugeführt werden, um die Netzwerkbildung unter erschwerten Bedingungen zu erleichtern und die Netzwerkdichte zu erhöhen. Sie werden aus folgender Gruppe ausgewählt:

Nanopartikel; Nanopartikel von Mono-, Oligo- und Polysacchariden; mikrokristalline Cellulose; oberflächenbehandelte mikrokristalline Zellulose; Polysaccharid-Mikrokristallite; Stärke-Mikrokristallite; mineralische Mikro- und Nanokristallite wie beispielsweise Bornitrid, Sorbitol-Derivate, insbesondere 3,4-dimethyl-dibenzyliden-Sorbitol; Titanoxid; Calciumcarbonat; Nanoclays; Mischungen von Elementen dieser Gruppe.

2. Keimstabilisatoren

Keimstabilisatoren können der Mischung der netzwerkfähigen Polysaccharide in mindestens einem der Schritte d) bis f) zugeführt werden, um insbesondere bei hochkonzentrierten Fluiden netzwerkfähiger Poysaccharide das Kristallitwachstum zu unterdrücken und somit bei der Unterkühlung eine hohe Kristallit-Keimzahl zu erhalten, was für die Netzwerkbildung bei tiefen Weichmachergehalten $WM_0$ nach dem Mischen mit dem Fluid der vorliegenden Polysaccharide von Bedeutung ist. Im Allgemeinen werden als Keimstabilisatoren stark verzweigte Polysaccharide verwendet, welche keine Gelbildung zeigen oder erst nach Tagen oder Wochen nur sehr schwache Gele bilden. Beispiele sind Glycogen, Amylopektin oder Agaropektin. Vorzugsweise werden Amylopektine mit einem Blue-Value von kleiner als 0.08 und/oder mit einer lod-Affinität von kleiner als 0.7g/100g eingesetzt.

### 3. Additive

Additive können in mindestens einem der Schritte a) bis g) zur Verbesserung der Verarbeitbarkeit, zur Beeinflussung der Netzwerkbildung und zur Modifikation der Produkteigenschaften mit Anteilen in Gew.% von 0.01% bis 10%, vorzugsweise von 0.02% bis 7%, noch bevorzugter von 0.03% bis 5% zugeführt werden. Unter anderem können Additive und Hilfsstoffe, welche bei der Herstellung von Thermoplastischer Stärke dem Stand der Technik entsprechen, auch für Polysaccharid-Netzwerke eingesetzt werden. Additive werden insbesondere aus folgender Gruppe von Stoffen ausgewählt:

Lebensmitteladditive, insbesondere Antioxidantien und Lebensmittel-Stabilisatoren; Glycerinderivate; Mono-, Di- und Triglyceride und deren Stearate; Glycerinmonostearat; Mono-, Di-, oder Triglyceride von Speisefettsäuren; Polyglycerinester, insbesondere der Speisefettsäuren; Polyethylen-glycole; Polyethylenglycolester, insbesondere der Speisefettsäuren; Lecithine; nichtionische und ionische Netzmittel und Tenside; Emulgatoren; Komplexbildner; Amylose-Komplexbildner; Na-2-stearoyl-Lactat; aliphatische Alkohole; Fettsäuren, insbesondere Stearinsäuren, aliphatische und aromatische Ester; Pyridin; Zucker; Zuckerester, insbesondere Zuckerester der Sepeisefettsäuren; Fette; Fettsäurenester; Wachse, insbesondere vegetarische Wachse wie CarnaubaWachs, Candelillawachse, Japanwachs, Ouricurywachs, Gagelstrauchwachs, Jojobawachs; Polyolefinwachse; Naturharze; Schellack; Chitin; Kollagen; Casein; Mono- und Oligosaccharide; Dextrane; Proteine; Peptide; Polypeptide, insbesondere pflanzliche Polypeptide; Cellulose, Cellulosederivate, insbesondere hydroxypropylierte Cellulose; Hydrocolloide, insbesondere Alginate, Carrageenane, Galactomannane, Glucomannane; Farbstoffe; als Lebensmittel verwendbare Stoffe; Aromastoffe; Mischungen von Elementen dieser Gruppe.

### 4. Füllstoffe

Füllstoffe können in mindestens einem der Schritte a) bis g), zugeführt werden, um Eigenschaften des Werkstoffs zu modifizieren und/oder die spezifischen Rohstoffkosten per Kilogram zu reduzieren. Allgemein kommen Füllstoffe in Frage, welche entsprechend dem Stand der Technik in der Kunststoff- und Biokunststofftechnik eingesetzt werden, insbesondere werden sie werden aus folgender Gruppe ausgewählt:

Mineralien, insbesondere Titandioxid, Talkum, Clays; Holzmehl; Lignin; Fasern, insbesondere Naturfasern wie Baumwolle, Hanffasern, Flachs, Ramie, Jutefasern; Russ; Glas; Glasfasern; Clays; native Stärke; inhibierte Stärke; vernetzte Stärke; Stärke mit einem Amylose-Gehalt von mehr als 40%; Mischungen von Elementen dieser Gruppe.

### 5. Polymere

Polymere können dem Verfahren in mindestens einem der Schritte a) bis c), f) und g) oder nach g) und vor h) zugesetzt werden, um Mischungen oder Blends von Polysaccharid-Netzwerken mit diesen Polymeren zu erhalten, wodurch die Eigenschaften der Netzwerke für bestimmte Anwendungen modifiziert und/oder verbessert werden können. Der Anteil der Polymere in Gew.% beträgt 5% - 95%, vorzugsweise 10% - 80%, noch bevorzugter 15% - 75%, am bevorzugtesten 20% - 70%. Dabei kann das Polysaccharid-Netzwerk sowohl als Matrix als auch als in der Polymer-Matrix dispergierte Phase vorliegen. Besonders geeignete Polymere sind biologisch abbaubare Polymere, jedoch kommen auch nicht biologisch abbaubare Polymere in Frage. Die Polymere werden bevorzugt aus folgender Gruppe ausgewählt:

Homo- oder Copolymere von aliphatischen Hydroxysäuren; aliphatische Polyester von Bicarboxylsäuren und aliphatischen Diolen, deren Laktone und Laktide, insbesondere Polyepsiloncaprolacton, Polyvinylacetate, Polyhydroxybutyrat, Polyhydroxybutyratvalerat, Polymilchsäure; Polyvinylalkohole, mit Acrylaten oder Methylacrylaten oder Anhydriden modifiziert, dessen Copolymere mit Vinylacetat, Ethyloxazolin oder Vinylpyrridin, und teilweise veresterte; Polyvinylacetate und teilhydrolysierte; Ethylen/Vinylalkohol Copolymere und modifizierte Formen davon, beispielsweise mit modifizierten Endgruppen, insbesondere mit Fettsäure-Endgruppen; Cellulose; Celluloseacetate und Cellulosenitrate, regenerierte Cellulose, Alkylcellulose, Carboxymethylcellulose, Cellulosepropionat, Cellulosebutyrat; Ethylen/Acrylsäure Copolymer; Polyethylen, dessen Vinyl-Copolymere, Polypropylen; Polyether, insbesondere Polyoxymethylen, Polyoxyethylen, Polyoxypropylen, Polyphenylenoxid; Polyamid; Polyacrylonitrile; Polyurethane; Polyester/Polyurethan Copolymere; Polyester/Polyamid Copolymere; Polyglycolide; hydrophie Polymere, insbesondere Polyvinylpyrrolidon, Polyoxazoline; Gelatine; Proteine, insbesondere Zein, Gluten, Casein Protein; Schellak; Chitin; Chitosan; Elastomere, insbesondere Naturkautschuk, Latex; Elemente dieser Gruppe, die Anhydrid-, insbesondere Maleinsäureanhydridgruppen enthalten und daher als Phasenvermittler eingesetzt werden können; Mischungen von Elementen dieser Gruppe.

### 6. Treibmittel

Treibmittel können dem Verfahren in mindestens einem der Schritte a) bis g), zugeführt werden, um geschäumte Produkte zu erhalten, wobei es sich dabei sowohl um geschäumte Polysaccharid-Netzwerke, als auch um geschäumte Polysaccharid-Polymer Blends handeln kann. Als Treibmittel können dem Stand der Technik entsprechende Treibmittel für Polymerschäume verwendet werden, bevorzugt werden sie aus folgender Gruppe ausgewählt:

Physikalische Treibmittel wie $CO_2$, $N_2$, n-Pentan, Wasser, wasserbindende Materialien wie Fasern von Ramie, Flachs, Hanf, Jute, Sisal, Baumwolle, Cellulose, wasserbindende Materialien wie Lehm, Silicagel, Gele, Sephadex, Zeolithe; chemische Treibmittel, insbesondere Azodikarbonid, OBSH (p, p-oxybisbenzolsulfonylhydrazide), TSSC (p-Toluolsulfonylsemicarbazide, THT (Trihydrazintriazid), Peroxide, Natriumbikarbonat; Mischungen von Elementen dieser Gruppe.

### 7. Spezifische Zusatzstoffe

Für spezifische Anwendungen kommt eine grosse Palette von Zusatzstoffen in Frage. Sie können für spezifische Anwendungen von Stärke-Gelen aus den verschiedensten Werkstoff- oder Stoffklassen ausgewählt werden. Es können spezifische Zusatzstoffe eingesetzt werden, die sich während dem Verfahren inert verhalten, so können beispielsweise magnetisierbare Partikel zugemischt und vor der Netzwerkbildung durch Magnetfelder ausgerichtet werden, um danach in erwünschter Orientierung fixiert zu bleiben, oder spezifische Zusatzstoffe, die sich beim Gebrauch der Netzwerke veränderen, nach einer einstellbaren Zeit oder unter Einwirkung eines auslösenden Faktors, oder spezifische Zusatzstoffe die sich während dem Verfahren verändern, ausgelöst beispielsweise durch Temperatur oder Druck, durch Einwirkung chemischer Stoffe, mechanischer Wellen, elektromagnetischer Felder und Wellen, insbesondere durch stehende Wellen, durch Bestrahlung wie Elektronenbeschuss, insbesondere durch Einwirkung von Laserstrahlung oder fokussierter Wellen, oder spezifische Zusatzstoffe oder Kombinationen von inerten oder sich während dem Verfahren verändernden Zusatzstoffen, die zusammen mit den genannten oder weiteren Einwirkungsmöglichkeiten oder Kombinationen dieser Einwirkungsmöglichkeiten eine gezielte dreidimensionale Modulation oder ein dreidimensionales Muster der Netzwerkeigenschaften erzeugen, die Netzwerkeigenschaften innerhalb eines Formkörpers ortsabhängig einstellen lassen, sodass z.B. Schichten oder anders definierte Bereiche unterschiedlicher Netzwerkeigenschaften entstehen, insbesondere es ermöglichen, dass definierte Zonen von sich voneinander un-terscheidenden Netzwerkeigenschaften entstehen, die das Spektrum von hohen oder höchsten Netzwerkdichten bis hin zu niederen oder verschwindenden Netzwerkdichten zulassen, oder insbesonderst es ermöglichen, dass definierte Hohlräume oder Hohlraumsysteme entstehen, sodass beispielsweise ein dreidimensionales Abbild eines Organs entsteht, worauf sich Stammzellen kultivieren lassen, die während der Formkörper durch gesteuerte Prozesse allmählich verschwindet, zu einem bezüglich der Form und immunologischer Eigenart massgeschneiderten implantierbaren lebendigen Organ heranwachsen.

### 8. Spezielle Zusätze

Durch spezielle Zusätze von gummiartigen Stoffen, insbesondere von Hydrokolloiden, kann die Zähigkeit der Gele massiv verbessert werden, indem der als eine separate Phase in der Polysaccharid-Matrix vorliegende spezielle Zusatz Spannungsspitzen auffangen kann. Die speziellen Zusätze werden vorzugsweise aus der folgenden Gruppe ausgewählt:

Galactomannane, wie Guar-Gummi oder Johannisbrotkernmehl; Pectine, insbesondere Rhamnogalakturonane und Protopektine; Dextrane; Xanthan; Zymosan; Hydrokolloide aus Meeresalgen, wie Alginate, Agar-Agar, Agarose, Carrageen und Carrageenane; Furcellaran; Hydrokolloide aus Flechten, wie Lichenine und Isolichenine, oder Hydrokolloide als Exsudate aus Hölzern, wie Tragant (Astragalus Gummi), Karaya-Gummi, Gummi arabicum, Kutira-Gummi; Inulin; Latex; Chitin; Chitosan; Kollagen; Casein; Mischungen von Elementen dieser Gruppe.

Um optimale Ergebnisse zu erhalten, ist eine möglichst feine Verteilung dieser Phase in der Matrix entscheidend. Bei gleichem Anteil an speziellem Zusatz ist der Gewinn an Zähigkeit entscheidend von dessen Verteilung in der Matrix und der Partikelgrösse abhängig. Dies wird einerseits ermöglicht, indem der spezielle Zusatz als möglichst feines Pulver vorbereitet wird, andererseits indem dieser Zusatz vorgängig gequollen wird und dann zu der vorliegenden Stärke im nativen Zustand bei geringem Weichmachergehalt zugegeben wird. Durch die bei der Mischung wirkenden Scherkräfte werden die gequollenen weichen Partikel des speziellen Zusatzes durch die harten nativen Stärkekörner fragmentiert und gemalen, sodass sich eine entsprechend fein verteilte Phase der speziellen Zusätze erhalten lässt.

Die Bedingungen der Zumischung der speziellen Zusätze zur Einstellung einer hochdispersen Phase der speziellen Zusätze sind:

A. Die speziellen Zusätze weisen einen Weichmachergehalt in Gew.% im Zeitpunkt der Zuführung von 5% - 90%, vorzugsweise von 11% - 90%, noch bevorzugter von 18% - 90%, insbesondere von 26% - 90%, am bevorzugtesten von 33% - 90% auf. Bevorzugt wird Wasser als Weichmacher beziehungsweise Quellmittel verwendet.

B. Der Mittelwert der Partikelgrössenverteilung der speziellen Zusätze bei 5% bis 20% Wassergehalt liegt im Bereich 150µm - 0.1µm, vorzugsweise im Bereich 100µm - 0.1µm, noch bevorzugter im Bereich 50µm - 0.1µm, insbesondere im Bereich 10µm - 0.1µm, am bevorzugtesten im Bereich 5 µm - 0.1µm.

C. Die speziellen Zusätze werden zu vorliegenden Polysacchariden im nativen, pregelatiniserten, teilweise oder ganz plastifizierten Zustand in mindestens einem der Schritte a) bis c) zugegeben, vorzugsweise im Schritt a), während der Weichmachergehalt der vorliegenden Polysaccharide in Gew.% zu diesem Zeitpunkt im Bereich 1% - 50%, vorzugsweise im Bereich 1% - 30%, noch bevorzugter im Bereich 1% - 20%, insbesondere im Bereich 1% - 15%, am bevorzugtesten im Bereich 1 % - 12% liegt.

Durch optimale Verfahrensweise kann ein spezieller Zusatz als hochdisperse Phase in der Matrix dispergiert werden, wobei die mittlere Grösse dieser Phase im Bereich 50µm - 0.07µm, vorzugsweise im Bereich 20µm - 0.07µm, noch bevorzugter im Bereich 7µm - 0.07µm, insbesondere im Bereich 3µm - 0.07µm, am bevorzugtesten im Bereich 1µm - 0.07µm liegt.

Das Zumischen der speziellen Zusätze unter den angegebenen Bedingungen ist neben der Herstellung von schlagzähen Polysaccharid-Netzwerken auch für die Herstellung von Thermoplastischer Stärke (TPS) mit verbesserter Zähigkeit vorteilhaft, wodurch die Eigenschaften von TPS für verschiedene Anwendungen verbessert werden und sich daher deren Anwendungsmöglichkeiten verbessern lassen.

Strukturtyp von Stärke-Netzwerken

Durch geeignete Prozessführung kann erreicht werden, dass die sich bildenden Kristallite bei Raumtemperatur bevorzugt eine A-Struktur aufweisen. Dieser Strukturtyp zeigt gegenüber dem bei Raumtemperatur stabilen B-Strukturtyp bei gleicher Luftfeuchtigkeit eine massiv reduzierte Wasseraufnahme, wodurch sich ein günstigeres Sorptionsverhalten ergibt. Der bei Raumtemperatur metastabile A-Strukturtyp kann durch kinetische Kontrolle eingefroren und so auch bei Raumtemperatur erhalten werden. Eine weitere Möglichkeit zur Einstellung des A-Strukturtyps ist gegeben durch eine Wärmebehandlung, wobei der B-Strukturtyp in den gewünschten A-Strukturtyp umgewandelt wird. Die notwendige Temperatur, die nur kurzzeitig angewendet werden muss, liegt oberhalb von 100°C.

**Parameter**

$T_{L0}$ — Minimale Temperatur, bei der sich die netzwerkfähigen Polysaccharide lösen

$T_{LR}$ — Temperatur der Rekristallisation der netzwerkfähigen Polysaccharide im thermodynamischen Gleichgewicht nach dem Lösen bei $T_{L0}$

$T_{L\ddot{U}}$ — Überhitzungstemperatur $T_{L\ddot{U}} > T_{L0}$

$T_{LM}$ — Temperatur, bei der der metastabile Zustand des unterdrückten Keimwachstums für 10 Sekunden aufrechterhalten werden kann

$\Delta T_{LU}$ — Unterkühlung $\Delta T_{LU} = T_{LR} - T_{LM}$

$T_{L1}$ — Temperatur der Lösung beim Einmischen des zweiten oder dritten Fluids in das erste Fluid, $T_{LM} < T_{L1} < T_{L\ddot{U}}$, insbesondere $T_{LM} <= T_{L1} < T_{LR}$

$T_1$ — Temperatur des ersten Fluids vor der Zuführung des zweiten oder dritten Fluids

$T_M$ — Temperatur während des Mischvorgangs

$T_3$ — Temperatur am Ende des Mischvorgangs

$T_K$ — Temperatur zu Beginn der Netzwerkbildung

$\Delta t_d$ — Zeitdauer des Ueberführens in Schritt d)

$\Delta t_e$ — Zeitdauer des Ueberführens in Schritt e)

$v_d$ — Aufheizgeschwindigkeit in Schritt d)

$v_e$ — Aufheizgeschwindigkeit in Schritt e)

$Z_K$ — Anzahl der Keime im dritten Fluid bei $T_{L1}$

$Z_N$ — Zahl der Fremdkeime in der Mischung von erstem und zweitem oder dritten Fluid

$Z$ — Zahl der bei der Netzwerkbildung aktiven Keime

$C_{PN}$ — Konzentration der netzwerkfähigen Polysaccharide im zweiten oder dritten Fluid

$C_{PNM}$ — Konzentration der netzwerkfähigen Polysaccharide in der Mischung

$C_{Sta}$ — Konzentration des Keimstabilisators im ersten Fluid

$C_{StaM}$ — Konzentration des Keimstabilisators in der Mischung

$C_N$ — Konzentration des Fremdnukleierungsmittels in der Mischung

$WM_d$ — Weichmachergehalt in Schritt d)

$WM_1$ — Weichmachergehalt der vorliegenden Polysaccharide zu Beginn des thermoplastischen Verfahrens

$WM_2$ — Weichmachergehalt der Mischung nach Zugabe des zweiten oder dritten Fluids

$WM_3$ — Weichmachergehalt am Ende des Mischvor-

gangs

WM$_0$ Weichmachergehalt bei der Netzwerkbildung

W$_0$ Wassergehalt bei der Netzwerkbildung

W$_1$ Wassergehalt nach Quellung eines Films mit W$_0$ in Wasser

G$_d$ Schergeschwindigkeit in Schritt d)

G$_g$ Schergeschwindigkeit in Schritt g)

p$_w$(T) Wasserdampfdruck bei der Temperatur T

N$_0$/V$_0$ Netzwerkdichte nach erfolgter Netzwerkbildung

DP Mittlerer Polymerisationsgrad

CL Mittlere Kettenlänge (Anzahl Monomereinheiten unverzweigter Kettensegmente)

Q$_b$ Durchschnittlicher Verzweigungsgrad: Anzahl Mole der verzweigten Monomereinheiten/Anzahl Mole der gesamten Monomereinheiten (für Stärke: Anzahl Mole der verzweigten α-Glucan-Einheiten/Anzahl Mole der gesamten α-Glucan-Einheiten)

BV Blue-Value

IA Iod-Affinität [g/100g]

M$_w$ Gewichtsmittel der Molekulargewichtsverteilung

T$_g$ Glasumwandlungstemperatur

Die Weichmacher- und Wassergehalte beziehen sich jeweils auf vorliegende und netzwerkfähige Polysaccharide, d.h. auf Polysaccharide, welche konstituierende Bestandteile des Netzwerks sind. Ein Netzwerk enthaltend beispielsweise 10g vorliegende Stärke, 3g netzwerkfähige Stärke, 11 g Wasser, 2g Glycerin, 7g Zucker und 5g eines Zusatzes weist daher einen Weichmachergehalt WM$_0$ von 100*(11+2)/(11+2+10+3) = 50% und einen Wassergehalt von 100*11/(11+10+3) = 45.8% auf.

Beispiele

Weitere Vorteile, Merkmale und Anwendungsmöglichkeiten der Erfindung ergeben sich aus der folgenden Beschreibung nicht einschränkend aufzufassender Beipiele im Zusammenhang mit der erfindungsgemässen Herstellung von Netzwerken auf Polysaccaridbasis und deren Blends.

Es zeigen:

Fig. 1:   das Quellverhalten von Stärke-Gel als Funktion des Wassergehaltes W$_0$

Fig. 2:   den Einfluss des Gehalts von netzwerkfähiger Stärke auf die Quellung

Fig. 3:   die Abhängigkeit der Zug-Bruchfestigkeit vom Wassergehalt gequollener Stärke-Gele

Fig. 4:   ein Beispiel für die Aufbereitung von netzfähiges Polysaccharid enthaltenden Lösungen

Fig. 5:   ein Beispiel für die kontinuierliche Aufbereitung von netzwerkfähiges Polysaccharid enthaltenden Lösungen

Fig. 6:   ein Beispiel für die Herstellung von Polysaccharid-Netzwerken

Fig. 7:   ein Beispiel für die Herstellung von Polysaccharid-Netzwerken

Eigenschaften

Beispiel 1

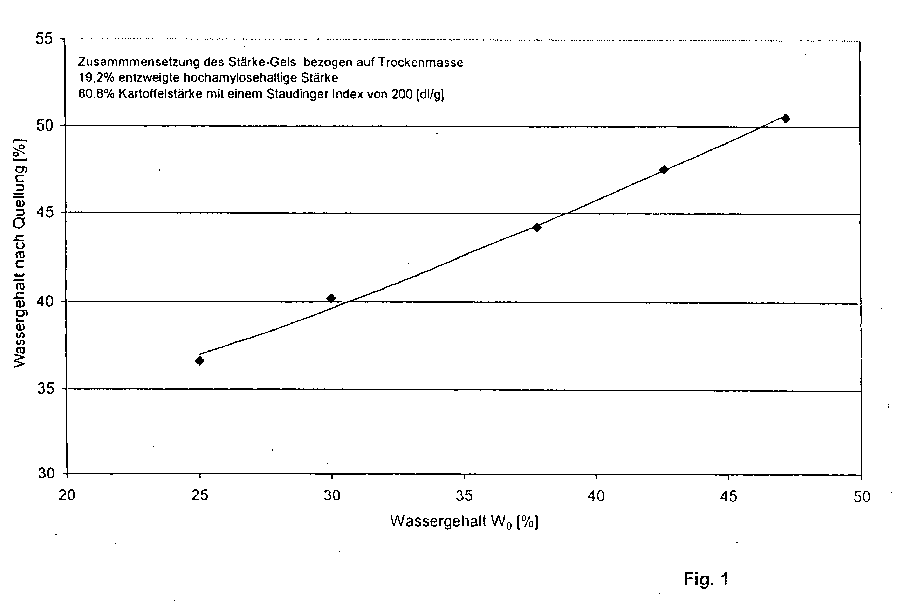

Fig. 1 zeigt das Quellverhalten von Stärke-Gel als Funktion des Wassergehalts W$_0$. Die entsprechenden Stärke-Gele wurden mit dem Wassergehalt W$_0$ hergestellt, wobei keine weiteren Weichmacher eingesetzt wurden. Als vorliegende Stärke wurde plastifizierte Kartoffelstärke verwendet, als netzwerkfähige Stärke eine entzweigte hochamylosehaltige Stärke. Nach der Herstellung des Netzwerks wurde der Wassergehalt W$_0$ konstant gehalten. Der Wassergehalt eines Films des Netzwerks nach der Quellung (24 Stunden in Wasser) wurde ausgehend von Stärke-Gel mit diesem Wassergehalt W$_0$ ermittelt. Der Verlauf der Kurve zeigt, dass der Wassergehalt nach der Quellung mit dem Wassergehalt W$_0$ deutlich abnimmt, d.h. um Stärke-Gel mit reduzierter Wasseraufnahme zu erhalten, ist die Herstellung des Gels mit möglichst tiefem Wassergehalt W$_0$ entscheidend.

Beispiel 2

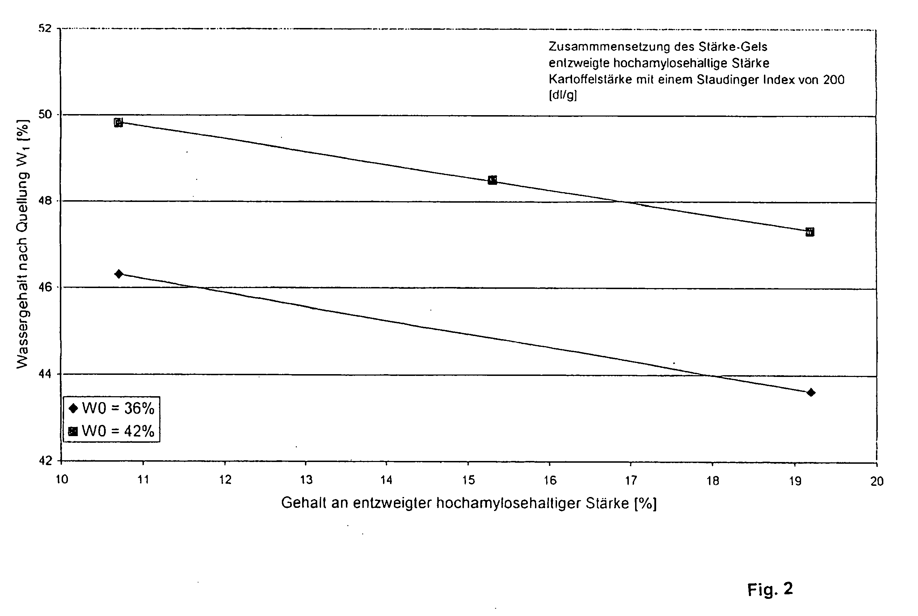

Den Messwerten von Fig. 2 liegt dieselbe Verfahrensweise und Messmethode wie bei Fig. 1 zugrunde, jedoch wurde hier der Gehalt an netzwerkfähiger Stärke variiert, um dessen Einfluss zu untersuchen. Die Resultate zeigen, dass der Wassergehalt nach der Quellung mit einer Zunahme des Gehalts an netzwerkfähiger Stärke abnimmt. Ebenso ist der Einfluss des Wassergehaltes W$_0$ ersichtlich.

Beispiel 3

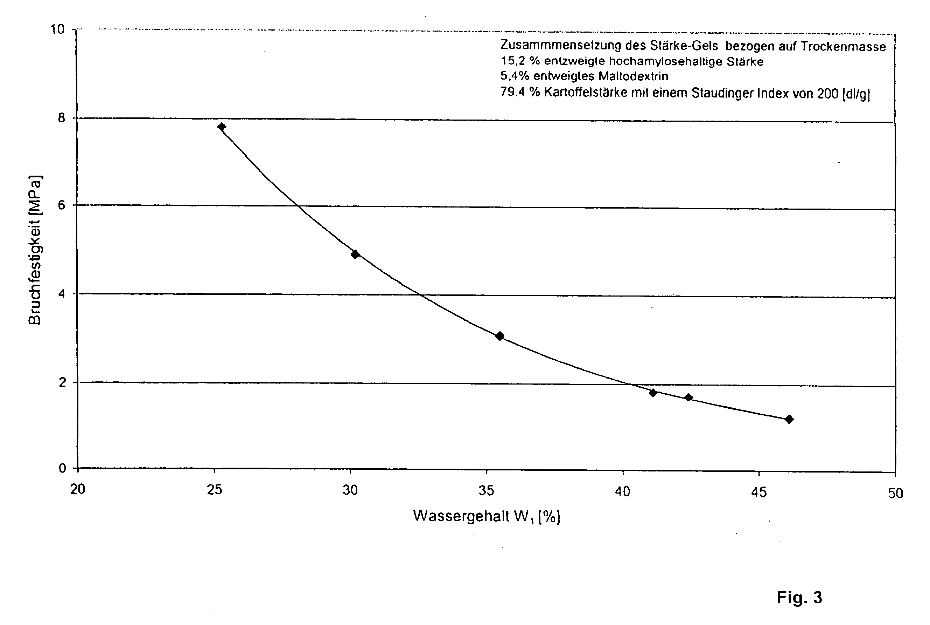

Fig. 3 zeigt die Bruchfestigkeit von Stärke-Netzwerken als Funktion des Wassergehalts W$_1$ (Wassergehalt nach erfolgter Quellung in Wasser). Als vorliegende Stärke wurde plastifizierte Kartoffelstärke, als netzwerkfähige Stärke eine Mischung von entzweigtem Maltodextrin und entzweigter hochamylosehaltiger Stärke eingesetzt, wobei die Komponenten vor dem Zusammenführung zur Gesamtmischung separat gelöst und unterkühlt wurden. Mit abnehmendem Wassergehalt W$_1$ der gequollenen Netzwerke steigt die Bruchfestigkeit deutlich an, bis auf rund 8MPa bei W$_1$ = 25%. Dies ist ein erstaunliches Resultat, das durch Ausschöpfung der Möglichkeiten des erfindungsgemässen Verfahrens erhalten werden konnte. Bisherige Werkstoffe auf Basis von Thermoplastischer Stärke haben Festigkeiten in diesem Bereich, sie lösen sich jedoch in Wasser vollständig auf. Mit erfindungsgemässen Stärke-Netzwerken konnten nun selbst nach vollständiger Quellung in Wasser typische TPS-Festigkeiten erhalten werden. Hier zeigen sich die Vorteile und Möglichkeiten von Netzwerken ganz deutlich. Die unterschiedlichen Quellgrade beziehungsweise Wassergehalte $W_1$ wurden durch eine Variation des Wassergehalts $W_0$ der Netzwerke bei und nach der Netzwerkbildung erhalten. Tiefe Wassergehalte $W_1$ wurden dabei durch tiefe Wassergehalte $W_0$ erhalten.

Beispiel 4

Erfindungsgemässe Stärke-Netzwerke konnten wie beschrieben, mittels vorliegenden Stärken, welche keine Gele oder Netzwerke bilden können, mit Stärken, welche zwar kristallisieren, jedoch für sich ebenfalls keine Gele bilden, mittels des vorgeschlagenen Verfahrens erhalten werden, wobei hierfür eine Heterokristallisation der Komponenten ausschlaggebend ist. Als Beispiel wurde für eine solche vorliegende, nicht-gelierfähige Stärke Waxy-Mais in nativer Form mit 15% gelöstem entzweigtem Maltodextrin gemischt und die Mischung unter hohen Schergeschwindigkeiten plastifiziert. Der Wassergehalt der Mischung $W_0$ bei und nach erfolgter Netzwerkbildung war 41%. Nach anschliessender Quellung in Wasser von Proben in Form von Filmen wurde ein Wassergehalt $W_1$ von 49% erhalten. Damit konnte gezeigt werden, dass ein Netzwerk vorlag.

Beispiel 5

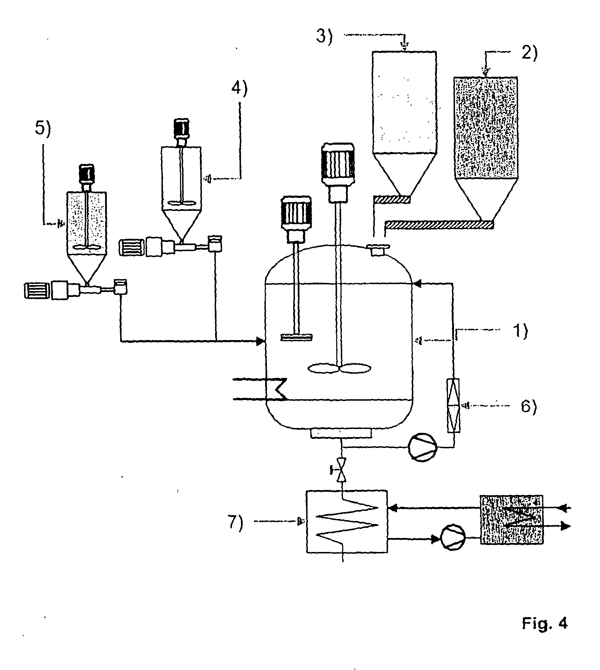

Im Figur 4 ist schematisch ein Beispiel zur Herstellung von Lösungen von netzwerkfähigen Polysacchariden dargestellt. Dabei werden zwei netzwerkfähige Polysaccharide, beispielsweise eine entzweigte hochamylosehaltige Stärke 2) und ein entzweigtes Maltodextrin 3), in einen Mischreaktor 1) sowie zwei vorgängig temperierte Weichmacher 4) und 5) zudosiert. Zusätzlich kann ein Keimstabilisator wie Glycogen zudosiert werden. Die Komponenten werden im Mischreaktor 1) gemischt und aufgeheizt.

Die Mischzeit und die Temperatur richten sich nach der Auslegung und oder Art der Anlage und der angeforderten Eigenschaften am Endprodukt. Beispielsweise nach einer Zeit von 1min und bei einer Temperatur von 180°C, wird die Mischung über einen statischen Mischer 6) mittels einer Pumpe dispersiv gemischt. Durch diese Massnahmen wird eine Lösung von zwei netzwerkfähigen Polysacchariden hergestellt, die je nach Zeit und Temperatur verschiedene Zustände haben kann. Die Art und Qualität der Mischungsgüte der Lösung richtet sich nach den anvisierten Applikationen bzw. Anforderungen an das herzustellende Polysaccharid-Netzwerk. Die vorbereitete netzwerkfähige Polysaccharidlösung (drittes Fluid) kann danach beispielsweise mittels eines Wärmetauschers 7) abgekühlt oder unterkühlt und so mittels einer gewünschten Keimzahl dem nachfolgenden Prozessschritt f) zugeführt werden.

Analog kann auch ein einzelnes netzwerkfähiges Polysaccharid oder können mehr als zwei Polysaccharide verarbeitet werden. Alternativ können mehrere netzwerkfähige Polysaccharide jeweils entsprechend diesem Verfahren aufbereitet, vor dem Wärmetauscher zusammengeführt werden oder jeweils nach dem Durchlauf durch den Wärmetauscher einzeln im Schritt f) dem ersten Fluid zugeführt werden.

Je nach Art und/oder Auslegung der Anlagenkomponenten und/oder der Fahrweise und/oder der Mischungsverhältnisse und/oder der Zusammensetzung kann dieser Prozess kontinuierlich geführt werden. In diesem Fall würde nach der Aufbereitung über den statischer Mischer der Stofffluss direkt über den Wärmetauscher geführt und für den nächsten Verfahrensschritt bereitgestellt.

Beispiel 6

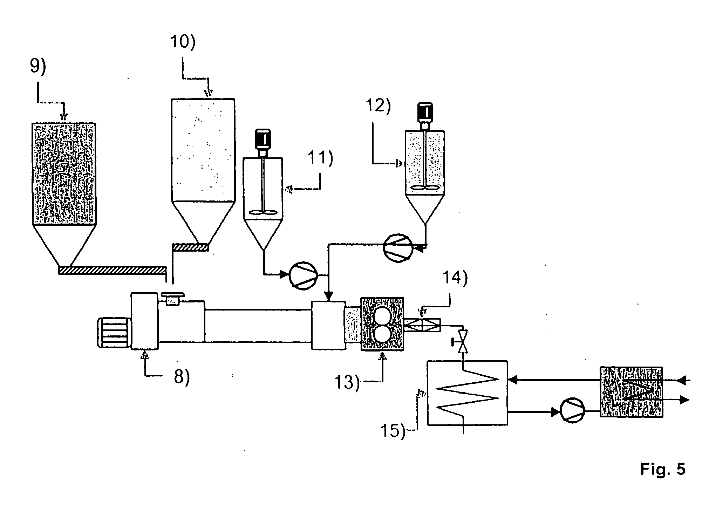

Im Figur 5 wird ein Beispiel zur kontinuierlichen Herstellung von netzwerkfähiger Polysaccharidlösung dargestellt. Die netzwerkfähigen Polysaccharide 9) und 10) werden kontinuierlich in den Pumpextruder 8) eindosiert. Nach einer ersten Förderzone werden die Weichmacher 11) und 12) ebenfalls kontinuierlich eindosiert. Der Pumpextruder führt die Komponenten einer Zahnradpumpe 13) zu. Die Zahnradpumpe baut den benötigten Druck auf, um die Komponenten über einen statischen Mischer 14) zu pumpen, wo bei beispielsweise 200°C eine dispersive Mischung stattfindet und die Polysaccharide unter Scherung innerhalb von Sekunden gelöst werden. Danach wird diese Lösung über einen Wärmetauscher 15) kontrolliert abgekühlt, beziehungsweise unterkühlt, wonach sie dem Verfahrensschritt f) zugeführt werden kann. Durch diese Verfahrensvariante können insbesondere hohe Konzentrationen von netzwerkfähigen Polysacchariden in Lösungen überführt werden.

Analog können mehrere netzwerkfähige Polysaccharide gemeinsam verarbeitet werden oder alternativ können jeweilige Polysaccharide entsprechend diesem Verfahren einzeln aufbereitet, vor oder nach dem Wärmetauscher zusammengeführt, oder einzeln dem Verfahrensschritt f) zugeführt werden.

Beispiel 7

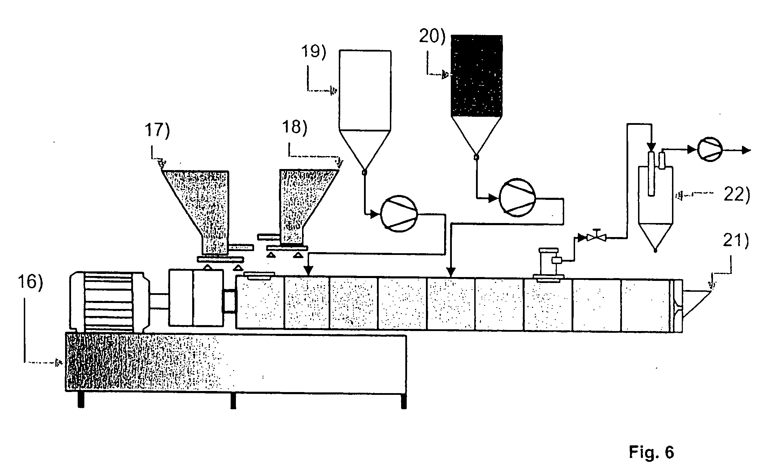

Gemäss Figur 6 werden zwei vorliegende Polysaccharide 17) und 18) kontinuierlich in einen Aufbereitungsextruder 16) dosiert. Nach einer kurzen Förderstrecke wird ein Weichmacher 19) zudosiert. Die vorliegenden Polysaccharide und der Weichmacher werden mittels geeigneter Schneckengeometrie, Drehzahl und Temperaturführung zu einem ersten Fluid aufbereitet. Danach wird die beispielsweise entsprechend Beispiel 1 oder 2 aufbereitete netzwerkfähige Polysaccharidlösung 20) in den Aufbereitungsextruder zudosiert und danach mit dem ersten Fluid gemischt. Nach der Mischung können mittels Entgasungstechnik 22) Weichmacheranteile entfernt werden. Nachfolgend wird der notwendige Druck aufgebaut, um die Mischung über ein Formwerkzeug 21) kontinuierlich zu formen.

Um Blends von Polysaccharid-Netzwerk mit Polymeren zu erhalten, kann beispielsweise durch einen Seitenextruder ein plastifiziertes Polymer nach der Entgasung der Polysaccharidmischung zugeführt und nachfolgend mit dieser zu einem Blend gemischt werden. Ein Schaum kann erhalten werden, wenn ein geeignetes Treibmittel dem Prozess zugegeben wird.

Beispiel 8

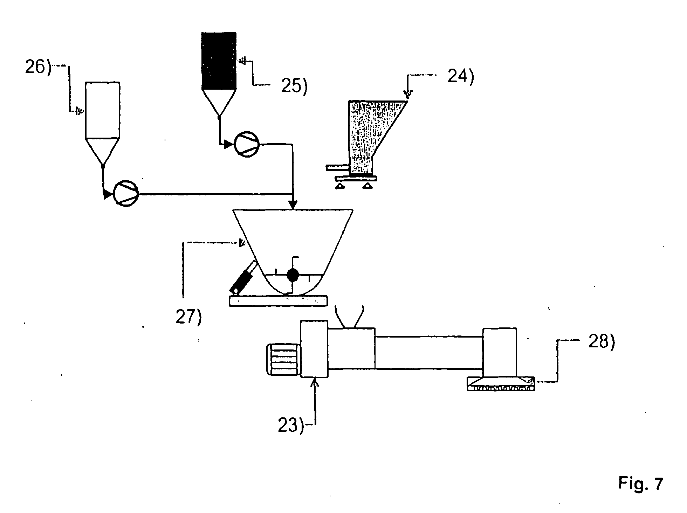

In Figur 7 wird im Unterschied zur Figur 6 die Mischung batchweise aufbereitet. Ein vorliegendes Polysaccharid 24) und ein Weichmacher 26) werden einem Mischer zudosiert und gemischt. Danach wird die entsprechende Menge an netzwerkfähiges Polysaccharid enthaltender Lösung 25) ebenfalls im Mischer zudosiert und die Gesamtmischung gemischt. Danach wird diese Gesamtmischung mittels eines Formextruders 23) und über eine Formgebungseinrichtung 28) in eine gewünschte Form gebracht.

**Bezugszeichenliste**

| 1 | Mischreaktor |
|---|---|
| 2 | Dosiereinrichtung für netzwerkfähiges PS A |
| 3 | Dosiereinrichtung für netzwerkfähiges PS B |
| 4 | Dosiereinrichtung für WM1 |
| 5 | Dosiereinrichtung für WM2 |
| 6 | Dispergiereinrichtung |
| 7 | Wärmetauscher |
| 8 | Pumpextruder |
| 9 | Dosiereinrichtung für netzwerkfähiges PS A |
| 10 | Dosiereinrichtung für netzwerkfähiges PS B |
| 11 | Doisiereinrichtung für WM1 |
| 12 | Dosiereinrichtung für WM2 |
| 13 | Zahnradpumpe |
| 14 | Statischer Mischer |
| 15 | Wärmetauschereinrichtung |
| 16 | Aufbereitungsextruder |
| 17 | Dosiereinrichtung für vorliegendes PS C |
| 18 | Dosiereinrichtung für vorliegendes PS D |
| 19 | Dosiereinrichtung für WM3 |
| 20 | Dosiereinrichtung für netzwerkfähiges PS-Lösung |
| 21 | Formgebungseinrichtung |
| 22 | Entgasungseinrichtung |
| 23 | Formgebungsextruder |
| 24 | Dosiereinrichtung für vorliegendes PS E |
| 25 | Dosiereinrichtung für netzwerkfähiges PS-Lösung |
| 26 | Dosiereinrichtung für WM3 |
| 27 | Mischer |
| 28 | Formgebungseinrichtung |
| PS.. | Polysaccharid |
| WM.. | Weichmacher |

**Patentansprüche**

1. Verfahren zum Herstellen eines Netzwerks auf Polysaccharidbasis, **dadurch gekennzeichnet, dass** das Netzwerk aus einer Gesamtmischung von mindestens einem vorliegenden Polysaccharid und mindestens einem netzwerkfähigen Polysaccharid durch Homo- und/oder Heterokristallisation gebildet wird, wobei netzwerkfähige Polysaccharide einen Verzweigunsgrad $Q_b$ kleiner 0.01 aufweisen; diese beiden Komponenten getrennt und individuell vorbereitet werden, wobei einerseits die vorliegenden Polysaccharide plastifiziert werden und die netzwerkfähigen Polysaccharide gelöst werden; und die Komponenten in diesem Zustand nach ihrem Zusammenführen zur Gesamtmischung molekulardispers gemischt werden.

2. Verfahren nach Anspruch 1, **dadurch gekennzeichnet, dass** das Verfahren folgende Schritte in mindestens einer Verfahrenszone aufweist:

   a) Zuführen jeweils eines vorliegenden Polysaccharids;
   b) Einwirken jeweils eines ersten Weichmachers auf das jeweils eine vorliegende Polysaccharid;
   c) Überführen des jeweils einen vorliegenden Polysaccharids in jeweils ein erstes Fluid, wobei jeweils eine erste Mischung gebildet wird;
   d) Überführen jeweils eines netzwerkfähigen Polysaccharids in jeweils ein zweites Fluid durch Einwirken jeweils eines zweiten Weichmachers;
   e) Überführen des jeweiligen zweiten Fluids in ein jeweiliges drittes Fluid;
   f) Einführen des jeweiligen zweiten Fluids aus Schritt d) oder des jeweiligen dritten Fluids aus Schritt e) in eine der jeweiligen ersten Mischungen aus den Schritten a) bis c);
   g) Vereinigen der jeweiligen Mischungen aus den Schritten a) bis f) zu einer Gesamtmischung;
   h) Bilden eines Polysaccharid-Netzwerkes aus der in Schritt g) gebildeten Gesamtmischung, insbesondere durch Homokristallisation zwischen jeweiligen Makromolekülen der jeweils mindestens einen netzwerkfähigen Polysaccharide untereinander und/oder durch Heterokristallisation zwischen diesen jeweiligen Makromolekülen und jeweiligen Makromolekülen der jeweils mindestens einen vorliegenden Polysaccharide, nachdem die Schritte a) bis g) vollzogen sind.

3. Verfahren nach Anspruch 2, **dadurch gekennzeichnet, dass** in mindestens einem der Schritte d) bis g), insbesondere nach Schritt f) und vor Schritt h), also vor der Netzwerkbildung, ein Weichmacher mindestens teilweise aktiv aus dem Verfahren entfernt wird.

4. Verfahren nach einem der vorangehenden Ansprüche, **dadurch gekennzeichnet, dass** in Schritt d) das netzwerkfähiges Polysaccharid enthaltende zweite Fluid überhitzt wird.

5. Verfahren nach einem der vorangehenden Ansprüche, **dadurch gekennzeichnet, dass** in Schritt e) das netzwerkfähiges Polysaccharid enthaltende dritte Fluid unterkühlt wird.

6. Verfahren nach einem der vorangehenden Ansprüche, **dadurch gekennzeichnet, dass** in mindestens einem der Schritte a) bis g) mindestens einmal ein Fremdnukleierungsmittel zugeführt wird.

7. Verfahren nach einem der vorangehenden Ansprüche, **dadurch gekennzeichnet, dass** in Schritt e) kurz vor Schritt f) das netzwerkfähiges Polysaccharid enthaltende dritte Fluid mit Ultraschall behandelt wird.

8. Verfahren nach einem der vorangehenden Ansprüche, **dadurch gekennzeichnet, dass** dem Verfahren in mindestens einem der Schritte a) bis g) mindestens ein Zusatz zugeführt wird.

9. Verfahren nach einem der vorangehenden Ansprüche, **dadurch gekennzeichnet, dass** das vorliegende Polysaccharid eine vorliegende Stärke und das netzwerkfähige Polysaccharid eine netzwerkfähige Stärke enthält, das vorliegende Polysaccharid eine vorliegende Stärke und das netzwerkfähige Polysaccharid eine netzwerkfähige Stärke ist.

10. Verfahren nach einem der vorangehenden Ansprüche, **dadurch gekennzeichnet, dass** die Schritte a), b), c), f) und g) entfallen und somit weitere Netzwerke basierend auf netzwerkfähigen Polysacchariden, insbesondere netzwerkfähigen Stärken, erhalten werden, wobei der Weichmachergehalt $WM_0$ der Netzwerke nach erfolgter Herstellung ohne nachträgliche Trocknung in Gew.% im Bereich 10% - 83% liegt.

11. Verfahren nach einem der vorangehenden Ansprüche, **dadurch gekennzeichnet, dass** der Weich-machergehalt $WM_0$ der Netzwerke nach erfolgter Herstellung ohne nachträgliche Trocknung in Gew.% im Bereich 10% - 97% liegt.

12. Verfahren nach einem der vorangehenden Ansprüche, **dadurch gekennzeichnet, dass** nach dem Schritt g) und vor, während oder nach dem Schritt h) ein Schritt h1) zum Formen des Gesamtgemisches durchgeführt wird, dass während des Schrittes h1) die Netzwerkbildung durch einen spezifischen Zusatz beeinflusst wird.

13. Verfahren nach einem der vorangehenden Ansprüche, **dadurch gekennzeichnet, dass** auf den Schritt h) oder h1) oder mit dem Schritt h1), ein Schritt h2) erfolgt, wobei das Polysaccharid-Netzwerk ganz oder teilweise vernetzt ist und durch eine Wärmebehandlung, insbesondere durch eine Wärmebehandlung in Kombination mit einer Variation des Weichmachergehalts $WM_0$, vorzugsweise des Wassergehalts, modifiziert wird, insbesondere getrocknet wird und/oder der kristalline Anteil in den A-Strukturtyp umgewandelt wird.

14. Netzwerk auf Polysaccharidbasis, insbesondere auf Stärkebasis, **dadurch gekennzeichnet, dass** das Netzwerk durch ein Verfahren gemäss einem der vorangehenden Ansprüche 1 bis 13 hergestellt wird und dass das Netzwerk transparent ist.

15. Netzwerk auf Polysaccharidbasis nach Anspruch 14, **dadurch gekennzeichnet, dass** das Netzwerk mit mindestens einem Polymer zu einem Blend gemischt ist.

16. Netzwerk auf Polysaccharidbasis nach Anspruch 14 oder 15, **dadurch gekennzeichnet, dass** das Netzwerk nach erfolgter Herstellung ohne nachträgliche Trocknung einen Weichmachergehalt $WM_0$ in Gew.% aufweist, der im Bereich 10% - 83% liegt.

17. Netzwerk auf Polysaccharidbasis nach einem der vorangehenden Ansprüche 14 bis 16, **dadurch gekennzeichnet, dass** das Netzwerk nach erfolgter Herstellung ohne nachträgliche Trocknung einen Weichmachergehalt $WM_0$ in Gew.% aufweist, der im Bereich 10% - 57% liegt.

18. Netzwerk auf Polysaccharidbasis nach einem der vorangehenden Ansprüche 14 bis 17, **dadurch gekennzeichnet, dass** bei dem nach der Herstellung erhaltenen Netzwerk mit $WM_0$, nachdem es in Form eines Films während 24 Stunden bei Raumtemperatur in Wasser gequollen wurde, die in diesem gequollenen Zustand bei Raumtemperatur gemessene Scherfestigkeit des Stärke-Netzwerks im Bereich 200kPa - 37MPa liegt.

19. Netzwerk auf Polysaccharidbasis nach einem der vorangehenden Ansprüche 14 bis 18, **dadurch gekennzeichnet, dass** bei dem nach der Herstellung erhaltenen Netzwerk mit $WM_0$, nachdem es in Form eines Films während 24 Stunden bei Raumtemperatur in Wasser gequollen wurde, die in diesem gequollenen Zustand bei 25°C gemessene Zugfestigkeit des Stärke-Netzwerks im Bereich 1MPa - 37MPa liegt.

20. Netzwerk auf Polysaccharidbasis nach einem der vorangehenden Ansprüche 14 bis 19, **dadurch gekennzeichnet, dass** das nach der Herstellung erhaltene Netzwerk in Form eines Films bei dem Weichmachergehalt $WM_0$, nachdem das Netzwerk während 24 Stunden bei Raumtemperatur in Wasser gequollen wurde, einen Wassergehalt in Gew.% von 10% - 60% aufweist.

21. Netzwerk auf Polysaccharidbasis nach einem der vorangehenden Ansprüche 14 bis 20, **dadurch gekennzeichnet, dass**, wenn das Netzwerk in Form eines Films von 0.3 mm Dicke, nachdem dieser Film getrocknet worden ist und in diesem Zustand in eine Atmosphäre mit maximal 43% Luftfeuchtigkeit bei Raumtemperatur gebracht und dort so lange aufbewahrt wurde, bis das Gewicht des Films sich nicht mehr änderte und der Film einen Wassergehalt von $W_{43}$ aufwies und, nachdem der Film darauf in eine Atmosphäre mit mindestens 90% Luftfeuchtigkeit bei Raumtemperatur gebracht wurde, bis das Gewicht sich nicht mehr ändert und der Film einen Wassergehalt von $W_{90}$ angenommen hat, die Differenz der Wassergehalte $W_{90}$ - $W_{43}$ in Gew.% 3% - 25% beträgt.

22. Netzwerk auf Polysaccharidbasis nach einem der vorangehenden Ansprüche 14 bis 21, **dadurch gekennzeichnet, dass** der kristalline Anteil, erhalten durch Separation des kristallinen Anteils vom amorphen Anteil der Weitwinkel-Röntgendiffraktionskurve einer Probe mit 7 bis 14 Gew.% Wasser, zwischen den Streuwinkeln $3° < 2\theta < 37°$, 15% - 100% beträgt.

23. Netzwerk auf Polysacharidbasis nach einem der vorangehenden Ansprüche 14 bis 22, **dadurch gekennzeichnet, dass** es als Film, Folie, Filament, Makro- oder Mikro-Faser, insbesondere als im Gel-Spinnverfahren hergestellte orientierte Fasern, als Schaum, Granulat, Pulver, in Form von Mikropartikeln, in sprühgetrockneter Form, in expandierter Form, als Formteil, Spritzgussteil, Stranggussteil, Profilgussteil, Tiefziehteil oder als Thermoformteil vorliegt.

24. Verwendung eines Netzwerks auf Polysaccharidbasis nach Anspruch 14, zur Herstellung eines Systems zur kontrollierten Abgabe und/oder Aufnahme spezifischer Stoffe, für die orale oder rektale Verabreichung von Wirkstoffen und/oder für die kontrollierte Abgabe und/oder Aufnahme von in der Landwirtschaft und im Garten benutzten Stoffen.

25. Verwendung eines Netzwerks auf Polysaccharidbasis nach Anspruch 14, zur Herstellung eines Implantats, z.B. einer Platte oder Schraube zur Fixierung von Knochenbrüchen, eines Stütznetzes, eines chirurgischen Werk- und Hilfsstoffes, beispielsweise einer Folie, eines Schlauches oder eines Fadens.

26. Verwendung eines Netzwerks auf Polysaccharidbasis nach Anspruch 14, im Lebensmittel-, Galenik-, Kosmetik-, Health Care -, Verpackungs- oder Agrarbereich, als Lebensmittel oder Lebensmittelzusatz, Gelatineersatz bei Gummibärchen und Geleeprodukten, als Bonbon, Ballaststoff, Fettersatz, als resistente Stärke und Prebiotikum, als Verdickungsmittel oder Zusatz bei Getränken, als Tablettierungshilfsstoff, Tablettensprengmittel, Glasur, Hart- und Weichkapsel, als Puder, Crème-Zusatz, Sonnenschutzmittel-Zusatz, Watte-Stäbchen, als Polystyrolschaum-Ersatz, Folie, biorientierte Folie, Verbundfolienkomponente, Papier-Laminat, als Ersatz von Cellulose und Zellstoff, Papier und Papiergebrauchsartikel, Einmal-Gebrauchskleidung, im Fast-Food Bereich, Geschirr und Besteck, Food-Tray, Trinkhalm, Becher, als Lebensmittelverpackung, in geschäumter Form als wärmedämmender Lebensmittelbehälter, als Kau-Knochen für den Hund, Einkaufs-Tragtasche, Kehrricht- und Kompostsack, als Mulch-Folie, Pflanzentopf, Golf-Tip, als Kinderspielzeug, Kauobjekt für zahnende Kleinkinder, in sprühgetrockneter oder in geschäumter Form als Feuchitgkeitsabsorber, zur Unterdrückung von Wolkenbildung oder Regen oder Gewitter in der Atmosphäre.

27. Verwendung eines Netzwerks auf Polysaccharidbasis nach Anspruch 14, zur Herstellung eines Systems zum Schutz vor Stoffen, als Schutz vor oxidativen oder geruchsintensiven oder giftigen Stoffen, als Schutzschicht von gegenüber Sauerstoff und Radikalen empfindlichen Wirkstoffen wie Arzneimitteln und Vitaminen, als Barriere- und/oder Membransystem zur Abschirmung und/oder zum Einschluss von Stoffen, als Barriere- und/oder Membransystem für die Nano,-Mikro- oder Makroenkapsulation, als Barriere- und/oder Membransystem für Nano- oder Mikroreaktoren und/oder Bioreaktoren.

28. Verwendung eines Netzwerks auf Polysaccharidbasis nach Anspruch 14, zur Herstellung eines Trägersystems, für den selektiven Transport chemischer Stoffe, für die chemische oder biochemische Analytik, die Gelpermeationschromatographie und die Gel-Elektrophorese von Molekülen, von Makromolekülen wie Proteinen, DNS und RNS sowie Polysacchariden und anderen hydrophilen Biomakromolekülen.

29. Verwendung eines Netzwerks auf Polysaccharidbasis nach Anspruch 14, als Substrat zum Herstellen von Geweben, Organteilen oder Organen im Bereich des Scaffold- oder Tissue-Engineering, als Substrat zur in-vitro Herstellung von Herzklappen.

30. Verwendung eines Netzwerks auf Polysaccharidbasis nach Anspruch 14, als Zusätze in Form einer hochdispersen Phase enthaltendes Netzwerk mit hoher Zähigkeit, wobei die mittlere Grösse dieser Phase im Bereich $50\mu m - 0.07\mu m$ liegt, als Polysaccharid- oder Stärke-Gummi, als Komponente von Gummi-Formulierungen, als die Dämpfungs-, Abrieb-, Haft- und Gleiteigenschaften von PKW- oder LKW-Reifen günstig beeinflussender Zuschlagstoff der entsprechenden Gummi-Formulierungen.

31. Verwendung eines Netzwerks auf Polysaccharidbasis nach Anspruch 14, als aus nachwachsenden, regenerierbaren und $CO_2$-neutralen Stoffen bestehender nachhaltiger Werkstoff, zur Substitution von herkömmlichen Kunststoffen.

## Claims

1. Method for the production of a polysaccharide-based network, **characterized in that** the network is formed from a total mixture of at least one available polysaccharide and at least one polysaccharide having network capabilities by homo- or heterocrystallization, polysaccharides having network capabilities possessing a degree of branching $Q_b$ of less than 0.01; these two components are prepared separately and individually, firstly the available polysaccharides being plasticized and the polysaccharides having network capabilities being dissolved; and, after their combination, the components are mixed in this state to give the total mixture in molecular disperse form.

2. Method according to Claim 1, **characterized in that** the method has the following steps in at least one zone of the method:

   a) feeding in of an available polysaccharide in each case;
   b) action of a first plasticizer in each case on the one available polysaccharide in each case;
   c) transfer of the one available polysaccharide in each case to a first fluid in each case, a first mixture being formed in each case;
   d) transfer of a polysaccharide having network capabilities in each case to a second fluid in each case by the action of a second plasticizer in each case;
   e) transfer of the respective second fluid to a respective third fluid;
   f) introduction of the respective second fluid from step d) or the respective third fluid from step e) into one of the respective first mixtures from steps a) to c);
   g) combination of the respective mixtures from steps a) to f) to give a total mixture;
   g) formation of a polysaccharide network from the total mixture formed in step g), in particular by homocrystallization between respective macromolecules of the respective at least one polysaccharide having network capabilities and/or by heterocrystallization between these respective macromolecules and respective macromolecules of the respective at least one available polysaccharide after steps a) to g) are complete.

3. Method according to Claim 2, **characterized in that** a plasticizer is actively removed at least partly from the method in at least one of the steps d) to g), in particular after step f) and before step h).

4. Method according to any of the preceding claims, **characterized in that** the second fluid containing polysaccharide having network capabilities is superheated in step d).

5. Method according to any of the preceding claims, **characterized in that** the third fluid containing polysaccharide having network capabilities is supercooled in step e).

6. Method according to any of the preceding claims, **characterized in that** a foreign nucleating agent is added at least once in at least one of the steps a) to g).

7. Method according to any of the preceding claims, **characterized in that** the third fluid containing polysaccharide having network capabilities is treated with ultrasound in step e) shortly before step f).

8. Method according to any of the preceding claims, **characterized in that** at least one additive is added to the method in at least one of the steps a) to g).

9. Method according to any of the preceding claims, **characterized in that** the available polysaccharide contains an available starch and the polysaccharide having network capabilities contains a starch having network capabilities, the available polysaccharide is an available starch and the polysaccharide having network capabilities is a starch having network capabilities.

**10.** Method according to any of the preceding claims, **characterized in that** the steps a), b), c), f) and g) are omitted and further networks based on polysaccharides having network capabilities, in particular starches having network capabilities, are thus obtained, the plasticizer content $WM_0$ of the networks after production is complete and without subsequent drying being, in % by weight, in the range 10% - 83%.

**11.** Method according to any of the preceding claims, **characterized in that** the plasticizer content $WM_0$ of the networks after production is complete and without subsequent drying, in % by weight, is in the range 10% - 97%.

**12.** Method according to any of the preceding claims, **characterized in that** a step h1) for moulding the total mixture is carried out after step g) and before, during or after step h), and **in that** the network formation is influenced by a specific additive during step h1).

**13.** Method according to any of the preceding claims, **characterized in that** a step h2) is effected after the step h) or h1) or with the step h1), the polysaccharide network being completely or partly crosslinked and being modified by a heat treatment, in particular by a heat treatment in combination with variation of the plasticizer content $WM_0$, preferably of the water content, in particular being dried and/or the crystalline fraction being converted into the A structure type.

**14.** Polysaccharide-based, in particular starch-based, network, **characterized in that** the network is produced by a method according to any of the preceding claims 1 to 13 and **in that** the network is transparent.

**15.** Polysaccharide-based network according to Claim 14, **characterized in that** the network is mixed with at least one polymer to give a blend.

**16.** Polysaccharide-based network according to Claim 14 or 15, **characterized in that**, after production is complete and without subsequent drying, the network has a plasticizer content $WM_0$ in % by weight which is in the range 10% - 83%.

**17.** Polysaccharide-based network according to any of the preceding Claims 14 to 16, **characterized in that**, after production is complete and without subsequent drying, the network has a plasticizer content $WM_0$ in % by weight which is in the range 10% - 57%.

**18.** Polysaccharide-based network according to any of the preceding Claims 14 to 17, **characterized in that**, in the case of the network obtained after the production with $WM_0$, after it was swollen in the form of a film for 24 hours at room temperature in water, the shear strength of the starch network, measured in this swollen state at room temperature, is in the range 200 kPa-37 MPa.

**19.** Polysaccharide-based network according to any of the preceding Claims 14 to 18, **characterized in that**, in the case of the network obtained after the production with $WM_0$, after it was swollen in the form of a film for 24 hours at room temperature in water, the tensile strength of the starch network, measured in this swollen state at 25°C, is in the range 1 MPa - 37 MPa.

**20.** Polysaccharide-based network according to any of the preceding Claims 14 to 19, **characterized in that** the network obtained after the production, in the form of a film, at the plasticizer content $WM_0$, has a water content in % by weight of 10% - 60% after the network was swollen for 24 hours at room temperature in water.

**21.** Polysaccharide-based network according to any of the preceding Claims 14 to 20, **characterized in that**, if the network is in the form of a film of 0.3 mm thickness, and after this film has been dried and was brought in this state into an atmosphere having a maximum relative humidity of 43% at room temperature and was stored there until the weight of the film no longer changed and the film had a water content of $W_{43}$, and after the film was then introduced into an atmosphere having a relative humidity of at least 90% at room temperature until the weight no longer changes and the film has assumed a water content of $W_{90}$, the difference between the water contents $W_{90}$ - $W_{43}$ in % by weight is 3% - 25%.

**22.** Polysaccharide-based network according to any of the preceding Claims 14 to 21, **characterized in that** the crystalline part, obtained by separation of the crystalline part from the amorphous part of the wide-angle X-ray diffraction curve of a sample with 7 to 14% by weight of water, between the scattering angles $3° < 2\theta < 37°$, is 15% - 100%.

**23.** Polysaccharide-based network according to any of the preceding Claims 14 to 22, **characterized in that** it is present as a film, sheet, filament, macro- or microfibre, in particular as oriented fibres produced in the gel spinning method, as foam, granules, powder, in the form of microparticles, in spray-dried form, in expanded form, as a shaped part, injection-moulded part, extruded part, continuously cast part or deep-drawn part or as a thermoformed part.

**24.** Use of a polysaccharide-based network according to Claim 14 for the production of a system for controlled release and/or uptake of specific substances, for oral or rectal administration of active substances and/or for controlled release and/or uptake of substances used in agriculture and in the garden.

25. Use of a polysaccharide-based network according to Claim 14 for the production of an implant, for example of a plate or screw for fixing bone fractures, of a support net, of a surgical engineering material and auxiliary, for example of a sheet, a tube or a filament.

26. Use of a polysaccharide-based network according to Claim 14 in the food, galenical, cosmetics, healthcare, packaging or agricultural sector, as food or a food additive, gelatin substitute in gummi bears and jelly products, as sweets, ballast or fat substitute, as resistant starch and prebiotic, as thickener or additive in beverages, as tabletting aid, tablet disintegrant, glaze or hard and soft capsules, as powder, cream additive, sunscreen additive or cotton bud, as a polystyrene foam substitute, sheet, bioriented sheet, composite sheet component or paper laminate, as a substitute for cellulose and pulp, paper and paper commodity articles, disposable utility wear, in the fast food sector, crockery and cutlery, food trays, drinking straws, cups, as food packaging, in foamed form as heat-insulating food containers, as chewing bones for dogs, carrier bags, rubbish and compost sacks, as mulch sheet, flower pots, golf tips, as children's toys, chewing objects for teething infants, in spray-dried or in foamed form as moisture absorbers, for suppression of cloud formation or rain or thunder in the atmosphere.

27. Use of a polysaccharide-based network according to Claim 14 for the production of a system for protection from substances, as protection from oxidative or odour-intensive or toxic substances, as a protective layer on active substances sensitive to oxygen and free radicals, such as medicaments and vitamins, as a barrier and/or membrane system for shielding and/or for enclosing substances, as a barrier and/or membrane system for nano-, micro- or macroencapsulation, as a barrier and/or membrane system for nano- or microreactors and/or bioreactors.

28. Use of a polysaccharide-based network according to Claim 14 for the production of a carrier system, for the selective transport of chemical substances, for chemical or biochemical analysis, gel permeation chromatography and gel electrophoresis of molecules, of macromolecules, such as proteins, DNA and RNA and polysaccharides and other hydrophilic biomacromolecules.

29. Use of a polysaccharide-based network according to Claim 14 as a substrate for the production of tissues, organ parts or organs in the area of scaffold or tissue engineering and as a substrate for the in vitro production of heart valves.

30. Use of a polysaccharide-based network according to Claim 14 as additives in the form of a network containing a highly disperse phase and having great toughness, the average size of this phase being in the range $50\mu m$ - $0.07\mu m$, or as a polysaccharide gum or starch gum, as a component of rubber formulations, as an additive advantageously influencing the damping, abrasion, adhesion and frictional properties of car or lorry tyres for the corresponding rubber formulations.

31. Use of a polysaccharide-based network according to Claim 14 as sustainable material consisting of renewable, regeneratable and $CO_2$-neutral substances, for the substitution of conventional plastics.

## Revendications

1. Procédé de fabrication d'un réseau à base de polysaccharides, **caractérisé en ce que** le réseau est formé à partir d'un mélange global d'au moins un polysaccharide de base et d'au moins un polysaccharide pouvant se réticuler grâce à la homo-et/ou hétérocristallisation pour lequel les polysaccharides pouvant se réticuler présentent un degré de ramification $Q_b$ inférieur à 0.01 ; ces deux composants sont préparés séparément et individuellement, les polysaccharides de base étant plastifiés et les polysaccharides pouvant se réticuler dissous ; et les composants sont mélangés dans cet état en dispersion moléculaire après leur réunion en un mélange global.

2. Procédé selon la revendication 1, se distinguant par le fait que le procédé se déroule selon les étapes suivantes dans l'une au moins des zones d'opération :

   a) Ajouter à chaque fois un polysaccharide de base
   b) Action d'un premier plastifiant sur le polysaccharide de base
   c) Transfert du polysaccharide de base dans un 1er fluide, formation d'un premier mélange ;
   d) Transfert du polysaccharide pouvant se réticuler dans un 2e fluide, sous l'action d'un 2e plastifiant ;
   e) Transfert du 2e fluide dans un 3e fluide ;
   f) Introduire le 2e fluide de l'étape d) ou le 3e fluide de l'étape e) dans l'un des premiers mélanges des étapes a) à c) ;
   g) Réunir les mélanges des étapes a) à f) en un mélange global ;
   h) Formation d'un réseau polysaccharide à partir du mélange global formé à l'étape g), en particulier grâce à l'homocristallisation entre les macromolécules d'un au moins des polysaccharides pouvant se réticuler et/ou grâce à l'hétérocristallisation entre ces macromolécules et les macromolécules d'un au moins des polysaccharides de base, une fois les étapes a) à g) achevées.

3. Procédé selon la revendication 2, **caractérisé en ce que** durant l'une au moins des étapes d) à g), en particulier après l'étape f) et avant l'étape h), donc avant la formation de réseau, un plastifiant soit éloigné au moins partiellement de manière active du procédé.

4. Procédé selon l'une des revendications précitées, **caractérisé en ce que** durant l'étape d) le $2^e$ fluide contenant le polysaccharide pouvant se réticuler est surchauffé.

5. Procédé selon l'une des revendications précitées, **caractérisé en ce que** durant l'étape e) le $3^e$ fluide contenant le polysaccharide pouvant se réticuler est superréfrigéré.

6. Procédé selon l'une des revendications précitées, **caractérisé en ce que** durant l'une au moins des étapes a) à g), on ajoute au moins une fois un agent de nucléation.

7. Procédé selon l'une des revendications précitées, se distinguant par le fait que durant l'étape e) juste avant l'étape f), le fluide contenant le polysaccharide pouvant se réticuler est traité aux ultrasons.

8. Procédé selon l'une des revendications précitées, se distinguant par le fait que l'on ajoute au procédé dans l'une au moins des étapes a) à g), au moins un adjuvant.

9. Procédé selon l'une des revendications précitées, se distinguant par le fait que le polysaccharide de base contient un amidon de base et le polysaccharide pouvant se réticuler un amidon pouvant se réticuler, que le polysaccharide de base est un amidon de base et le polysaccharide pouvant se réticuler un amidon pouvant se réticuler.

10. Procédé selon l'une des revendications précitées, se distinguant par le fait que les étapes a), b),c), f) et g) tombent et de ce fait d'autres réseaux basés sur des polysaccharides pouvant se réticuler, en particulier des amidons pouvant se réticuler sont conservés et que la teneur en plastifiant $WM_0$ des réseaux, une fois la fabrication réussie, se situe, sans séchage ultérieur, en poids % entre 10% - 83%.

11. Procédé selon l'une des revendications précitées, se distinguant par le fait que la teneur en plastifiant $WM_0$ des réseaux, une fois la fabrication réussie, se situe, sans séchage ultérieur, en poids % aux environs de 10% - 97%.

12. Procédé selon l'une des revendications précitées, se distinguant par le fait qu'après l'étape g) et avant, pendant ou après l'étape h) on exécute une étape h1) pour former le mélange global, que pendant l'étape h1) le formage de réseau est influencé par un adjuvant spécifique.

13. Procédé selon l'une des revendications précitées, se distinguant par le fait qu'après l'étape h) ou h1) ou avec l'étape h1), suit une étape h2), où le réseau polysaccharide est entièrement ou partiellement réticulé et modifié par un traitement thermique, en particulier par un traitement thermique combiné à une variation de la teneur en plastifiant $WM_0$, de préférence de la teneur en eau, est en particulier séché, et/ou la part cristalline est transformée dans le type de structure A.

14. Réseau à base de polysaccharides, en particulier à base d'amidon, se distinguant par le fait que le réseau est réalisé selon un procédé répondant à l'une des 1-13 revendications précitées, le réseau étant transparent.

15. Réseau à base de polysaccharides selon la revendication 14, se distinguant par le fait que le réseau est mélangé avec un moins un polymère pour forme un mélange.

16. Réseau à base de polysaccharides selon la revendication 14 ou 15, se distinguant par le fait que le réseau, une fois réalisé sans séchage ultérieur, présente une teneur en plastifiant $WM_0$ en poids% se situant entre 10% - 83%.

17. Réseau à base de polysaccharides selon l'une des revendications 14 à 16, se distinguant par le fait que le réseau, une fois réalisé sans séchage ultérieur, présente une teneur en plastifiant $WM_0$ en poids% se situant entre 10% - 57%.

18. Réseau à base de polysaccharides selon l'une des revendications 14 à 17, se distinguant par le fait que le réseau, une fois réalisé sans séchage ultérieur, avec $WM_0$, après avoir été gonflé dans l'eau sous forme d'un film pendant 24 heures à la température ambiante, la solidité de cisaillement du réseau-amidon, mesurée à la température ambiante dans cet état de gonflement, se situe entre 200kPa - 37Mpa.

19. Réseau à base de polysaccharides selon l'une des revendications 14 à 18, se distinguant par le fait que le réseau obtenu après la fabrication avec $WM_0$, après avoir été gonflé dans l'eau sous forme d'un film pendant 24 heures à la température ambiante, la résistance à l'étirement du réseau-amidon, mesurée à la température de 25°C dans cet état de gonflement, se situe entre 1 MPa - 37Mpa.

20. Réseau à base de polysaccharides selon l'une des revendications 14 à 19, se distinguant par le fait que le réseau obtenu après la fabrication sous forme d'un film avec une teneur en plastifiant $WM_0$ , après avoir été gonflé dans l'eau pendant 24 heures à la température ambiante, présente une teneur en eau en poids % de 10% - 60%.

21. Réseau à base de polysaccharides selon l'une des revendications 14 à 20, se distinguant par le fait que, lorsque le réseau formant un film épais de 0.3mm, une fois ce film séché et mis dans cet état dans une atmosphère comportant au maximum 43% d'humidité à la température ambiante et qu'il y soit conservé ainsi aussi longtemps jusqu'à ce que le poids du film ne change plus et que le film présente une teneur en eau de $W_{43}$ et que le film aura été mis ensuite dans une atmosphère avec au moins 90% d'humidité à la température ambiante jusqu'à ce que son poids ne change plus et qu'il ait pris une teneur en eau de $W_{90}$ , la différence des teneurs en eau $W_{90}$ - $W_{90}$ se situe en poids % entre 3% - 25%.

22. Réseau à base de polysaccharides selon l'une des revendications 14 à 21, se distinguant par le fait que la part cristalline, obtenue par la séparation de la part cristalline de la part amorphe de la courbe de diffraction radiographique à grand angulaire d'une épreuve, avec un poids en eau de 7 à 14%, se situe entre les angles de dispersion $3° < 20 < 37°$, 15% - 100%.

23. Réseau à base de polysaccharides selon l'une des revendications 14 à 22, se distinguant par le fait qu'il se présente comme film, feuille, filament, macro ou microfibre, en particulier comme fibres orientées, réalisées selon de procédé de filage des gels, comme mousse, granulat, poudre, sous forme de microparticules, sous forme séchée par atomisation, sous forme expansée, comme objet moulé, comme partie de moulage par injection, comme partie obtenue par coulée continue, par coulée profilée, par coulée d'emboutissage profond ou par thermoformage.

24. Utilisation d'un réseau à base de polysaccharides selon la revendication 14, pour réaliser un système de livraison et/ou de réception contrôlée de substances spécifiques, pour l'administration orale ou rectale de substances actives et/ou de livraison et/ou de réception contrôlée de substances utilisées dans l'agriculture et le jardin.

25. Utilisation d'un réseau à base de polysaccharides selon la revendication 14 pour réaliser un implant, par ex. une plaque ou une vis de fixation utilisés dans les fractures d'os, d'un réseau d'appui, d'un matériau ou d'une substance utilisés en chirurgie, par ex. un film, un tube ou un fil.

26. Utilisation d'un réseau à base de polysaccharides selon la revendication 14 dans le secteur alimentaire, les produits Galénic, le secteur de la cosmétique, de la santé, de l'emballage et le secteur agricole, comme denrée alimentaire ou comme adjuvant alimentaire, pour remplacer la gélatine dans les oursons en caoutchouc et les produits de gelée, comme les bonbons, comme lest, succédané de graisse, comme amidon résistant et comme prébiotique, comme agent épaississant ou adjuvant de boissons, comme substance aidant à la fabrication de tablettes, comme agent explosif de tablettes, de glaçage, comme capsules dures ou molles, comme poudre, adjuvant de crème, adjuvant de crèmes de protection solaire, de bâtonnets ouatés, de succédané à la mousse polystyrène, comme film, composants de films d'emballage, de papier laminé, comme succédané à la cellulose, au papier et aux articles usuels en papier, aux habits jetables, dans le secteur du fast-food, de la vaisselle et des couverts, du foodtray (les plateaux-repas), des chalumeaux ou paille pour boire, des gobelets, de l'emballage des denrées alimentaires, sous forme expansée comme les onteneurs de denrées alimentaires réduisant la chaleur, comme os à mâcher pour le chien, comme sac de commission, comme sac de poubelle, comme film servant de mulch ou de paillis, comme pot de fleur, de golf-tip, comme jouet d'enfant, d'anneaux de dentition pour les petits enfants, comme élément hygroscopique de forme expansée ou séché par atomisation, pour réprimer la formation de nuages ou de pluie ou d'orage dans l'atmosphère.

27. Utilisation d'un réseau à base de polysaccharides selon la revendication 14 pour fabriquer un système de protection contre certains substances, comme protection contre des substances oxydantes ou toxiques ou dégageant une forte odeur, comme couche de protection d'agents actifs sensibles à l'oxygène et aux radicaux comme les médicaments et les vitamines, comme barrière et/ou système de membranes pour envelopper et/ou séparer des substances, comme barrière et/ou système de membranes pour la nano, micro ou macro-encapsulation, comme barrière et/ou système de membranes pour les nano ou micro-réacteurs et/ou bioréacteurs.

28. Utilisation d'un réseau à base de polysaccharides selon la revendication 14 pour fabriquer un système de supports pour le transport sélectif de substances chimiques, pour l'analytique chimique ou biochimique, pour la chromatographie sur gel ou gelperméation et la gel-électrophorèse de molécules, de macromolécules comme les protéines, DNS et RNS ainsi que des polysaccharides et autre biomacromolécules hydrophiles.

29. Utilisation d'un réseau à base de polysaccharides selon la revendication 14 comme substrat pour réaliser des tissus, des parties d'organe ou des organes dans le secteur scaffold-ou tissue-engineering, comme substrat pour la fabrication de valvules cardiaques in vitro.

30. Utilisation d'un réseau à base de polysaccharides selon la revendication 14 comme adjuvants sous forme d'un réseau contenant une phase hautement dispersée avec une forte viscosité, la taille moyenne de cette phase se situant dans le secteur entre 50$\mu$m - 0.07 $\mu$m, comme caoutchouc de polysaccharide ou d'amidon, comme composant de formules du caoutchouc, comme adjuvant des différentes formules du caoutchouc ayant une excellente influence sur les qualités des pneus de voiture et de camion au point de vue amortissement, abrasion, adhérence et glissement.

31. Utilisation d'un réseau à base de polysaccharides selon la revendication 14 comme matériau durable, composé de substances renouvelables qui se régénèrent et sont neutres au $CO_2$, pouvant se substituer aux matériaux synthétiques usuels.

Zusammmensetzung des Stärke-Gels bezogen auf Trockenmasse
19,2% entzweigte hochamylosehaltige Stärke
80.8% Kartoffelstärke mit einem Staudinger Index von 200 [dl/g]

Wassergehalt nach Quellung [%]

Wassergehalt $W_0$ [%]

Fig. 1

Fig. 2

Fig. 3

Fig. 4

Fig. 5

Fig. 6

Fig. 7

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9428029 A
- US 5362777 A
- US 5382611 A
- US 5427614 A
- WO 9607693 A
- WO 9428063 A
- WO 9504104 A
- US 5801207 A
- US 5736586 A
- WO 9404600 A
- US 5415827 A
- US 5462980 A
- US 5360830 A
- US 5569692 A
- US 5384170 A
- WO 9631561 A

- WO 9748764 A
- US 5280055 A
- US 5314934 A
- US 5844023 A
- US 6348524 B
- US 6277899 B
- US 5874486 A
- US 5412005 A
- US 5334634 A
- US 5292782 A
- US 5262458 A
- US 5258430 A
- WO 9806755 A
- WO 9923118 A
- US 6117925 A